# FASCICULE DE BREVET EUROPEEN

(11) **EP 1 073 664 B1**
(45) Date de publication et mention de la délivrance du brevet: **23.10.2002**
(21) Numéro de dépôt: 99914616.0
(22) Date de dépôt: 20.04.1999
(51) Int. Cl.: C07F 9/32, A61K 31/66

(54) **DERIVES D'(ALPHA-AMINOPHOSPHINO) PEPTIDES ET COMPOSITIONS LES CONTENANT**
ALPHA-AMINOPHOSPHINOPEPTID-DERIVATE UND SIE ENTHALTENDE ZUSAMMENSETZUNGEN
(ALPHA-AMINOPHOSPHINO) PEPTIDESDERIVATIVE AND COMPOSITIONS CONTAINING SAME

(30) Priorité: 22.04.1998 FR 9805009
(43) Date de publication de la demande: 07.02.2001
(73) Titulaire: INSTITUT NATIONAL DE LA SANTE ET DE LA RECHERCHE MEDICALE (INSERM), 75654 Paris Cédex 13 (FR)
(72) Inventeur: ROQUES, Bernard, F-75014 Paris (FR); FOURNIE-ZALUSKI, Marie-Claude, F-75011 Paris (FR); CHEN, Xuixiong, F-92220 Bagneux (FR)
(74) Mandataire: Grosset-Fournier, Chantal Catherine
(86) Numéro de dépôt international: FR9900921
(87) Numéro de publication internationale: WO99054336

(56) Documents cités:
- EP-A- 0 053 902
- WO-A-96/33201
- WO-A-97/00261
- WO-A-98/18803
- CHACKALAMANNIL S ET AL: "HIGHLY POTENT AND SELECTIVE INHIBITORS OF ENDOTHELIN CONVERTING ENZYME" BIOORGANIC & MEDICINAL CHEMISTRY LETTERS, vol. 6, no. 11, 1996, pages 1257-1260, XP000676968
- CHEN H.: "Aminophosphinic inhibitors as transition state analogues of enkephalin-degrading enzymes: A class of central analgesics" PROCEEDINGS OF THE NATIONAL ACADEMY OF SCIENCES OF USA., vol. 95, no. 20, - 29 septembre 1998 pages 12028-12033, XP002104657 WASHINGTON US

## Description

La perception, la transmission et la régulation des influx nociceptifs sont sous la dépendance de plusieurs neurotransmetteurs endogènes. En 1975, Hugues et coll. (*Nature*, **258,** 577, 1975) ont mis en évidence les enképhalines, deux pentapeptides isolés primitivement de cerveaux de mammifères qui sont impliqués dans la transmission des influx douloureux.

Les enképhalines se lient à, au moins, deux classes de récepteurs : les sites opioïdes µ et δ (Pert, *Sciences,* **179,** 1011, 1973) dont les rôles et les localisations sont différents.

Leurs propriétés antinociceptives ont été démontrées par Belluzi et coll.. *Nature,* **260,** 625, 1976. Cependant, l'analgésie induite par administration d'enképhalines exogènes est très fugace, à cause de la métabolisation rapide de ces peptides. Des analogues d'enképhalines rendus résistants à la dégradation enzymatique par des modifications chimiques, ont été synthétisés, mais leurs effets secondaires sont analogues à ceux de la morphine.

Les enképhalines sont physiologiquement dégradées par deux types d'activités enzymatiques qui métabolisent *in vivo* Les enképhalines : l'endopeptidase neutre (EC 3.4.24.11, également désignée par EPN) qui coupe la liaison Gly³-Phe⁴ et l'aminopeptidase N (EC 3.4.11.2, également désigné par APN) qui coupe la liaison Tyr¹-Gly² (revue dans Roques et al., *Pharmacol*. *Rev*., **45,** 87-146, 1993).

On connait des prodrogues décrites dans le brevet européen EP-A- 0 487 620 et dans Fournié-Zaluski et al., *J. Med. Chem.,* **35,** 2473, 1992, qui possédent des activités analgésiques et antidépressives après administration intraveineuse ou par voie orale (Noble et al., *J. Pharm. Exp. Ther.,* **261,** 181, 1992 ; Baamonde et al., *Eur. J. Pharmacol*., **216,** 157, 1992).

Cependant, ces composés ne répondent pas au concept d'inhibiteurs mixtes, du fait de leur structure dans laquelle un inhibiteur d'APN et un inhibiteur d'EPN sont associés par un pont disulfure. Ces composés sont ensuite réduits par les réductases cérébrales et agissent chacun sur leur cible particulière.

D'après la demande de brevet WO 95/35302 et *Bioorganic & Médicinal Chemistry Letters,* Vol.6, No. 11, pp 1257-1260, 1996, on connaît certains dérivés de l'acide phosphinique possédant respectivement une activité inhibitrice de l'enzyme de conversion de l'endotheline (ECE) et une activité inhibitrice mixte de l'enzyme de conversion de l'angiotensine (ACE) et de l'endopeptidas neutre (EPN). Ces composés sont utiles dans le traitement de maladies cardiovasculaires.

Des dérivés d'(α-aminophosphino)peptides sont décrits dans la demande de brevet EP-A-1 009 750.

L'un des objets de l'invention est de fournir de nouveaux composés, qui se comportent comme de véritables inhibiteurs mixtes d'APN et d'EPN, capables d'inhiber conjointement les deux activités enzymatiques responsables de la dégradation des enképhalines et de manifester leurs propriétés pharmacologiques après injection intraveineuse ou sous-cutanée, ou par voie orale (per os).

Ces composés présentent certaines propriétés des substances morphiniques, en particulier l'analgésie, les effets bénéfiques sur le comportement (antidépresseurs, sédatifs, anxiolytiques, désinhibiteurs et promnésiques), et les effets périphériques (antidiarréiques, antitussifs, hypotenseurs, anti-inflammatoires...). De plus, un avantage de ces composés est qu'ils ne présentent aucun des effets néfastes des morphiniques (tolérance, dépendance physique et psychique, dépression respiratoire, stase intestinale...).

Ces composés peuvent également être utiles comme traitement de substitution dans la toxicomanie aux opioïdes.

La présente invention a pour objet des composés dérivés d'(α-aminophosphino)peptides de formule générale (I) 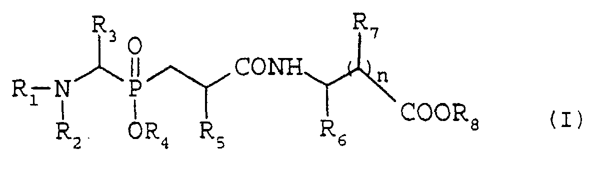 dans laquelle
- R₁ et R₂ représentent chacun un atome d'hydrogène ou bien R₁ et R_{2,} pris ensemble, forment un groupe insaturé de formule R'(R")C=, dans laquelle,
   - R' représente un groupe phényle monosubstitué en position 2 par un groupe hydroxy ou bien un groupe phényle disubstitué, en position 2, par un groupe hydroxy et, en position 4 ou 5, soit par un atome d'halogène soit par un groupe nitro, soit par un groupe hydroxy, soit par un groupe alcoxy -OR₉,
   - R" représente un groupe phényle, un groupe phényle substitué par 1 à 5 atomes d'halogène ou un groupe hétérocyclique aromatique,
par la suite, les termes R₉ et R₁₀, utilisés pour la définition des radicaux, représentent chacun un groupe alkyle de 1 à 6 atomes de carbone,
R₃ représente
- un atome d'hydrogène,
- un groupe alkyle ou un groupe alkényle de 1 à 6 atomes de carbone, ces deux derniers groupes pouvant être substitués par :
   - un groupe hydroxy ou un groupe alcoxy -OR₉,
   - un groupe phényle ou un groupe benzyle,
   - un groupe sulfanyle, un groupe alkylsulfanyle -SR₉ ou un groupe alkylsulfanyle oxydé sur l'atome de soufre -S(O)R₉,
   - un groupe amino, un groupe -NHR₉ ou -NR₉R₁₀, éventuellement oxydé sur l'atome d'azote ou,
   - un groupe guanidino H₂N-C(=NH)-NH-,
- un groupe cycloalkyle ou cycloalkylméthyle,
- un groupe phényle, un groupe benzyle, pouvant être substitués sur le groupe phényle par 1 ou 2 des substituants suivants :
   - un atome d'halogène,
   - une groupe hydroxy, un groupe alcoxy -OR₉,
   - un groupe alkylsulfanyle -SR₉ ou un groupe alkylsulfanyle oxydé sur l'atome de soufre,
   - un groupe amino ou un groupe -NHR₉ ou -NR₉R₁₀ éventuellement oxydé sur l'atome d'azote,
   - un groupe nitro,
   - un groupe phényle,
   - un groupe alkyle de 1 à 4 atomes de carbone,
- un groupe méthyle substitué par un groupe hétérocyclique aromatique ou saturé, les hétéroatomes pouvant être oxydés sous forme de. N-oxyde ou de S-oxyde,
R₄ représente
- un groupe -CH(X)-O-C(O)-Y, où X et Y représentent, indépendemment l'un de l'autre, un groupe R₉ ou un groupe phényle,
- un groupe -CH₂CH₂-S-C(O)-W, où W représente un groupe R₉ ou un groupe phényle,
R₅ représente
- un atome d'hydrogène,
- un groupe alkyle ou un groupe alkényle de 1 à 6 atomes de carbone, ces deux derniers groupes pouvant être substitués par :
   - un groupe hydroxy ou un groupe alcoxy -OR₉,
   - un groupe phényle ou un groupe benzyle,
   - un groupe sulfanyle, un groupe alkylsulfanyle -SR₉ ou un groupe alkylsulfanyle oxydé sur l'atome de soufre -S(O)R₉,
   - un groupe amino, un groupe -NHR₉ ou -NR₉R₁₀, éventuellement oxydé sur l'atome d'azote ou,
   - un groupe guanidino H₂N-C(=NH)-NH-,
- un groupe cycloalkyle ou cycloalkylméthyle,
- un groupe phényle, un groupe benzyle, pouvant être substitués sur le groupe phényle par 1 ou 2 des substituants suivants :
   - un atome d'halogène,
   - un groupe hydroxy, un groupe alcoxy -OR₉,
   - un groupe alkylsulfanyle -SR₉ ou un groupe alkylsulfanyle oxydé sur l'atome de soufre,
   - un groupe amino ou un groupe -NHR₉ ou -NR₉R₁₀ éventuellement oxydé sur l'atome d'azote,
   - un groupe nitro,
   - un groupe phényle,
   - un groupe alkyle de 1 à 4 atomes de carbone,
- un groupe méthyle substitué par un groupe hétérocyclique, les hétéroatomes pouvant être oxydés sous forme de N-oxyde ou de S-oxyde,
- R₆ et R₇ représentent indépendamment l'un de l'autre
   - un atome d'hydrogène,
   - un groupe alkyle ou alkényle de 1 à 6 atomes de carbone, pouvant être substitué par :
      > un groupe hydroxy ou un groupe alcoxy -OR₉,
      > un groupe sulfanyle, un groupe alkylsulfanyle -SR₉ ou un groupe alkylsulfanyle oxydé sur l'atome de soufre -S(O)R₉,
      > un groupe amino ou un groupe alkylamino -NHR₉,
      > un groupe guanidino H₂N-C(=NH)-NH- ou,
      > un groupe carboxy ou un groupe alkyloxycarbonyl -COOR₉,
   - un groupe phényle, un groupe benzyle, pouvant être substitués sur le groupe phényle par 1 ou 2 substituants suivants :
      > un atome d'halogène,
      > un groupe phényle,
      > un groupe hydroxy ou un groupe alcoxy -OR₉,
      > un groupe alkylsulfanyle -SR₉ ou un groupe alkylsulfanyle oxydé sur l'atome de soufre -S(O)R₉,
- R₆ et R₇ représentent ensemble un cycle saturé ou insaturé à 5 ou 6 chaînons, comprenant 1 ou 2 hétéroatomes pris parmi l'oxygène, le soufre et l'azote,
- R₈ représente,
   - un groupe alkyle ou alkényle de 1 à 6 atomes de carbone,
   - un groupe phényle, un groupe benzyle,
- n est égal à 0 ou 1,

Dans le cadre de l'invention les termes ci-après ont les significations suivantes :
- un groupe alkyle est une chaîne hydrocarbonée, saturée, linéaire ou ramifiée,
- un groupe alkényle est une chaîne hydrocarbonée linéaire ou ramifiée, comportant une insaturation,
- un groupe cycloalkyle est une chaîne hydrocarbonée cyclique comprenant 3 à 7 atomes de carbone,
- un groupe cycloalkylalkyle est un groupe cycloalkyle lié à un groupe alkyle, ce groupe alkyle comprenant 1 à 6 atomes de carbone,
- un groupe cycloalkylméthyl est un groupe cycloalkyle lié à un groupe méthyle,
- un groupe hétérocyclique est une chaîne hydrocarbonée cyclique, aromatique ou non, à 5 ou 6 chaînons, comprenant 1 ou 2 hétéroatomes pris parmi les atomes d'oxygène, de soufre ou d'azote,

Dans le cadre de l'invention, les atomes d'halogène sont préférentiellement le chlore et le fluor.
Lorsqu'un groupe phényle est substitué par un groupe phényle, il l'est alors préférentiellement en position 4 pour former un groupe biphényle (s'écrit également : [1,1'-biphényl]).

La présente invention a également pour objet les sels d'addition aux acides pharmacologiquement acceptables des composés de formule (I) pour lesquels R₁ et R₂ sont des atomes d'hydrogène.

Une catégorie de composés préférés selon l'invention sont ceux pour lesquels les radicaux de la formule (I) ont les significations suivantes :
- R₁ et R₂ représentent des atomes d'hydrogène,
- n est égal à 0,
- R₃ représente
   - un atome d'hydrogène,
   - un groupe alkyle de 1 à 6 atomes de carbone, pouvant être substitué par un groupe hydroxy, un groupe alcoxy -OR₉, un groupe sulfanyle ou un groupe alkylsulfanyle -SR₉,
   - un groupe phényle, un groupe benzyle pouvant être substitués sur le groupe phényle par un atome d'halogène, un groupe alkyle de 1 à 4 atomes de carbone, un groupe hydroxy, un groupe alcoxy -OR₉ ou un groupe alkylsulfanyle -SR₉,
- R₄ représente
   - un groupe -CH(X)-O-C(O)-Y, où X et Y représentent, indépendemment l'un de l'autre, un groupe R₉ ou un groupe phényle,
   - un groupe -CH₂CH₂-S-C(O)-W, où W représente un groupe R₉ ou un groupe phényle,
- R₅ représente
   - un groupe phényle, un groupe benzyle, pouvant être substitués sur le groupe phényle par un atome d'halogène, un groupe alkyle de 1 à 4 atomes de carbone, un groupe hydroxy, un groupe alcoxy -OR₉ ou un groupe alkylsulfanyle -SR₉,
   - un groupe biphénylméthyle,
- R₆ représente un groupe alkyle de 1 à 6 atomes de carbone, pouvant être substitué par un groupe hydroxy, un groupe alcoxy -OR₉, un groupe sulfanyle ou un groupe alkylsulfanyle -SR₉,
- R₈ représente
   - un groupe alkyle de 1 à 6 atomes de carbone,
   - un groupe phényle, un groupe benzyle.

Les composés de formule (I) peuvent possèder de 1 à 5 atomes chiraux sur le squelette et éventuellement de 1 à 3 atomes chiraux sur les différents groupements de R₃ à R₈. Les composés de l'invention peuvent exister sous différentes formes isomères optiques, à savoir sous forme d'énantiomères et de diastéréoisomères. La présente invention comprend ces différentes formes ainsi que leurs mélanges, y compris les mélanges racémiques.

Le carbone portant le radical R₆, lorsque R₆ est différent de l'atome d'hydrogène, est avantageusement de configuration absolue (*S*).

Les composés de l'invention de formule (I) peuvent être préparés selon les procédés décrits dans les annexes 1, 2 et 3.

Dans la description du procédé, les radicaux A₁ et A₂ ont les significations suivantes :
- A₁ représente un groupe biphénylméthyle, un groupe tert-butoxycarbonyle (Boc), un groupe benzyloxycarbonyle (Cbz ou Z) ou un groupe fluorénylméthoxycarbonyle (Fmoc),
- A₂ représente un groupe tert-butoxycarbonyle (Boc) ou un groupe fluorénylméthoxycarbonyle (Fmoc).

tBu, représente un groupe tertiobutyle et Bn représente un groupe benzyle.

Les composés de formule (Ia) et (Ib), qui sont des composés de formule (I) selon l'invention, sont préparés selon le procédé représenté en annexe 1. R₃, R₄, R₅, R₆, R₇, R₈, R', R" et n ont les significations données dans la formule (I). Selon ce procédé, on fait réagir un dérivé de l'acide hydroxyphosphinylpropanoïque de formule (IVb) avec un dérivé d'acide aminé de formule (V), en présence de benzotriazol-1-yloxy-tris(dimethylamino)phosphonium hexafluorophosphate (BOP) ou. bien de 1-éthyl-3-(3-diméthylaminopropyl)carbodiimide méthiodide (EDC), dans un solvant organique tel que le diméthylformamide. Le composé de formule (V) peut être utilisé sous forme de sel, tel que le sel de l'acide *p*-toluènesulfonique. Il est alors particulièrement avantageux d'opérer en présence d'une base telle qu'une aminé tertiaire comme la diisopropyléthylamine. Cette réaction permet l'obtention d'un composé de formule (III).

Le composé de formule (III) est utilisé pour préparer le composé de formule (II), dans lequelle les trois fonctions amine, phosphinate et carboxylate sont protégées simultanément. Le composé de formule (III) est traité dans un solvant organique tel que le tétrahydrofurane, par un alcool de formule R₄OH, en présence de *N*,*N*'-dicyclohexylcarbodiimide (DCC) et de 4-diméthylaminopyridine (DMAP) pour obtenir le composé de formule (II).
Alternativement, le composé de formule (III) est transformé en sel de cesium par action de carbonate de cesium. Ce sel obtenu est ensuite traité, dans un solvant organique tel que le diméthylformamide (DMF), par un dérivé halogène R₄Z, où Z représente un atome d'halogène tel que le brome, le chlore ou l'iode, pour obtenir le composé de formule (II).

Pour préparer le composé de formule (Ia), on procède à la déprotection de la fonction amine. Si A₂ est un groupe *tert*-butoxycarbonyl, la déprotection est effectuée dans un milieu acide doux, tel que l'acide formique. Si A₂ est un groupe fluorenylméthoxycarbonyl, la déprotection est effectuée dans un milieu basique tel que la pipéridine.

Pour préparer le composé de formule (Ib), on condense une cétone R'(R")C=O sur un composé de formule (Ia), ces cétones R'(R")C=O étant obtenues par réarrangement de Fries des esters correspondants R"CO₂R'.

Les composés de formule (V), dans laquelle n peut être égal à 0 ou 1, R₆, R₇ et R₈ prenant l'une des significations données dans la formule (I), représentent un α ou un β aminoacide naturel ou non. Ils peuvent être synthétisés selon les méthodes classiques bien connues de l'homme de l'art.

Les composés de formule (IVb) peuvent être préparés selon les procédés décrits dans les annexes 2 et 3.
Selon le schéma 1, annexe 2, on additionne un dérivé de l'acide phosphinique de formule (VIII) sur un dérivé de l'ester acrylique de formule (VII), en présence de

*N*,*O*-bis(triméthylsilyl)acétamide, sans solvant ou dans un solvant organique inerte tel que l'acétonitrile pour obtenir le composé de formule (VI). Si A₁ est un groupe biphényle méthyle ou benzyloxycarbonyle, une déprotection en milieu acide bromhydrique libère la fonction amine qui est ensuite reprotégée par un groupe *tert*-butoxycarbonyle ou un groupe fluorenylméthoxycarbonyle selon les procédés bien connus de l'homme de l'art, pour obtenir le composé de formule (IVa). L'obtention du composé de formule (IVb) à partir du composé de formule (IVa) est une saponification classique ou une hydrolyse en milieu acide.

Dans le cas où R₅ représente dans le produit final de formule (I) un groupe biphénylméthyle, on utilise, selon une variante, un composé de formule (VI), dans laquelle le radical R₅ représente tout d'abord un groupe (4-bromophényl)méthyle. On fait réagir ce composé obtenu avec de l'acide phénylboronique dans un solvant, tel qu'un mélange toluène/méthanol et en présence de tétrakis(triphénylphosphine)palladium et de carbonate de sodium pour obtenir le composé de formule (VI), dans laquelle R₅ est un groupe biphénylméthyl.

Le composé de formule (VIII) est obtenu par synthèse indirecte ou directe, représentée dans le schéma 2 de l'annexe 2 :
- par la voie indirecte, le procédé consiste à traiter de la diphénylméthylamine par de l'acide phosphoneux et à faire réagir le sel de diphénylméthylamine de l'acide phosphoneux obtenu, avec un aldéhyde R₃CHO dans de l'éthanol anhydre pour obtenir un composé de formule (IX). La fonction amine de ce composé de formule (IX) est éventuellement ensuite déprotégée dans l'eau acidifiée par un acide minéral, tel que l'acide chlorhydrique ou l'acide bromhydrique, puis le composé obtenu est traité par de l'oxyde de propylène et enfin acylé pour obtenir un composé de formule (VIII),
- par la voie directe, le procédé consiste à traiter le chlorhydrate de diphénylméthylamine par l'acide phosphoneux et un aldéhyde R₃CHO, cette réaction étant effectuée dans un mélange d'éthanol et d'eau au reflux.

Les composés de formule (VII) peuvent être obtenus par deux méthodes, qui sont représentées dans l'annexe 3, respectivement aux schémas 3 et 4 :
- selon le premier procédé (schéma 3), on fait réagir un halogénure, et de préférence un bromure, d'alkyle ou d'aralkyle R₅Z avec le triéthylphosphonoacétate, en présence d'hydrure de sodium pour obtenir un composé de formule (X) que l'on fait réagir sur du formaldéhyde, en présence de carbonate de potassium pour obtenir un composé de formule (VII).
- selon le second procédé (schéma 4), on obtient les composés de formule (VII) par réaction de Mannich sur un monoester d'un acide malonique de formule (XI).

Les composés de formule générale (I), ainsi obtenus, sont sous forme d'isomères, y compris sous forme d'énantiomères, de diastéréoisomères et de mélanges de ces différentes formes, y compris de mélanges racémiques.

Les composés de formule (I), optiquement purs, peuvent être obtenus par séparation par HPLC semi-préparative (Kromasil® C₈, 10 mm, 20 x 250 mm, acétonitrile-eau, 15 ml/min).

Les composés de formule (I), optiquement purs peuvent également être obtenus par dédoublement, à partir d'une amine chirale et du dérivé de l'acide phosphinique de formule (VIII), puis éventuellement par addition de Michaël diastéréosélective, en présence d'inducteurs chiraux, qui conduit aux composés de formule (VI), qui ont une stéréochimie parfaitement définie.

Les composés de formule (I), optiquement purs, peuvent enfin être obtenus pas dédoublement ou par résolution enzymatique des intermédiaires de synthèse (VI), (IVa) ou (IVb).

Les séparations envisagées précédemment peuvent être obtenues sur colonne semi-préparative Kromasil® C₈, 10 mm, 20x250 mm,15 ml/min) avec un mélange acétonitrile / eau.

Les exemples suivants ont pour but d'illustrer la préparation de quelques composés de l'invention. Les analyses élémentaires et les spectres RMN confirment les structures des composés obtenus.

Dans les noms des composés, le tiret "-" fait partie du mot, et le tiret "_" ne sert que pour la coupure en fin de ligne ; il est à supprimer en l'absence de coupure et ne doit être remplacé ni par un tiret normal ni par un espace.

Dans les exemples suivants, les acides aminés utilisés lors de ces synthèses ont une configuration absolue (*S*).

Concernant la nomenclature des composés décrits dans les exemples qui suivent, par convention, on n'indique pas la configuration du phosphore.

### Exemple 1 : Formiate de (R,S)-N-[3-[[2-(acéthylthio)éthoxy] (1-aminoéthyl)phosphinyl]-2-([1,1'-biphényl]-4-ylméthyl)-1-oxopropyl]-L-alaninate de phénylméthyle

### 1.1. 4-Bromo-α-(diéthoxyphosphinyl)benzènepropanoate d'éthyle

A une solution de 9 ml (45,4 mmol) de triéthylphosphono_ acétate dans 14 ml de diméthylformamide, on ajoute, à -10°C, 1,5 g (49,9 mmol) d'hydrure de sodium à 80%. Après 15 minutes, on ajoute, goutte à goutte 11,8 g (47,3 mmol) de bromure de (4-bromophényl)méthyle. Le mélange réactionnel est porté à une température voisine de 20°C et agité à cette température pendant la nuit. Le solvant est évaporé sous pression réduite. Le résidu est repris dans de l'acétate d'éthyle, lavé à l'eau, séché sur sulfate de sodium et filtré. Le solvant est évaporé sous pression réduite. On purifie le résidu par chromatographie sur gel de silice en éluant par un mélange heptane / acétate d'éthyle : 1 / 4. On récupère 7,8 g de produit sous forme d'huile. (rendement = 43,7%).

### 1.2. 4-Bromo-α-méthylènebenzènepropanoate d'éthyle

Un mélange de 5,5 g (14 mmol) de 4-bromo-α-(diéthoxy_ phosphinyl)benzènepropanoate d'éthyle, de 5,3 ml (71 mmol) de formaldéhyde et de 5,8 g (41,8 mmol) de carbonate de potassium est porté à reflux pendant 3 heures. On ajoute un mélange d'eau et d'éther diéthylique. La phase aqueuse est extraite avec de l'éther diéthylique. L'ensemble des phases organiques est lavé à l'eau, séché sur sulfate de sodium, filtré et évaporé sous pression réduite. Le résidu est distillé sous pression réduite.
On récupère 6,7 g de produit sous forme d'huile. (rendement = 75 %).

### 1.3. Acide [1-[(diphénylméthyl)amino]éthyl]phosphinique

Une solution de 10 g (45,5 mmol) de chlorhydrate de diphénylméthylamine et 4,8 ml (46,3 mmol) d'acide hypophosphoneux à 50% dans l'eau dans 100 ml d'un mélange eau / éthanol : 90 / 10, est porté au reflux. On ajoute goutte à goutte, 4 g (0,09 mmol) d'acétaldéhyde. Après addition, on maintient le reflux pendant une heure. Le précipité obtenu est filtré et lavé avec 100 ml d'eau et 100 ml d'acétone. On récupère 8 g de produit (rendement = 64 %).
Point de fusion : 236 °C.

### 1.4. Acide (1-aminoéthyl)phosphinique

Un mélange de 4,5 g (16,4 mmol) d'acide [1-[(diphénylméthyl) amino]éthyl]phosphinique et de 30 ml d'acide chlorhydrique 6N est porté à reflux pendant 2 heures. Deux phases apparaissent. Le mélange est mis à sec par évaporation sous vide à chaud puis repris dans 30 ml d'eau. On lave trois fois la phase aqueuse à l'éther diéthylique. La phase aqueuse, à nouveau évaporée, conduit au chlorhydrate de l'acide (1-aminoéthyl)phosphinique, qui est repris dans 15 ml d'éthanol absolu. On ajoute 8 ml d'oxyde de propylène, goutte à goutte, sous agitation, à 0°C. On observe la formation d'un précipité blanc abondant qui est filtré et lavé à l'éther diéthylique.
On récupère 1,7 g de produit (rendement = 95,3%).
Point de fusion : 222-224°C (décomposition).

### 1.5. Acide [1-[[(phénylméthoxy)carbonyl]amino]éthyl] phosphinique

A une solution de 1,7 g (15,6 mmol) d'acide (1-aminoéthyl) phosphinique dans 35 ml d'un mélange de dioxane et d'eau, en présence d'un équivalent de soude (624 mg), on ajoute, à 0°C, goutte à goutte, et simultanément une solution de 3,1 g (18,3 mmol) de chloroformiate de benzyle dans 9 ml de dioxane et, de manière à maintenir le pH de la réaction autour de 9, une solution de 1 g de soude dans 8,7 ml d'eau. A la fin de l'addition, le mélange est maintenu à une température voisine de 20°C pendant 2 heures. On lave ensuite la phase aqueuse par 3 fois 20 ml d'éther diéthylique. On acidifie, à 0°C, sous vive agitation, la phase aqueuse avec 11 ml d'une solution d'acide chlorhydrique 6N. Il se forme un précipité blanc abondant qui est filtré, lavé à l'eau et séché. On récupère 2,8 g de produit (rendement = 73,9%).
Point de fusion : 132-134°C.

### 1.6. Acide (R)-[1-[[(phénylméthoxy)carbonyl]amino]éthyl] phosphinique

A une solution de 13,2 g (54,3 mmol) de l'acide [1-[[(phényl_ méthoxy)carbonyl]amino]éthyl]phosphinique dans 45 ml de méthanol à reflux, on ajoute goutte à goutte une solution de 7 ml (54,3 mmol) de D(+)-α-méthylbenzylamine dans 3 ml de méthanol. Le solvant est distillé sous pression réduite. On recristallisation 3 fois le résidu dans un mélange d'acétate d'éthyle et isopropanol.
On récupère 6,2 g de sel.
A 6,2 g de ce sel on ajoute à température ambiante 51 ml d'acide chlorhydrique 1N. Après 15 minutes, la phase aqueuse est extraite avec de l'acétate d'éthyle. L'ensemble des phases organiques est lavé à l'eau, séché sur sulfate de sodium, filtré et évaporé sous pression réduite.
On récupère 4 g de produit sous forme d'un solide blanc (rendement = 60,6%).
Point de fusion : 134°C.
[α]²⁰_{D} = -45° (c=1, éthanol).

### 1.7. (R)-3-(4-Bromophényl)-2-[[hydroxy[1-[[(phénylméthoxy) carbonyl]amino]éthyl]phosphinyl]méthyl]propanoate d'éthyle

Une solution de 2,9 g (11,9 mmol) de l'acide
(*R*)-[1-[[(phénylméthoxy)carbonyl]amino]éthyl]phosphinique, 3,5 g (13 mmol) de 4-bromo-α-méthylènebenzènepropanoate d'éthyle et 6,2 ml de *N*,*O*-bis(triméthylsilyl)acétamide est porté à 60°C pendant 24 heures. Après refroidissement, on ajoute 60 ml d'un mélange d'acétate d'éthyle et d'eau (1 : 1 en volume). La phase aqueuse est extraite par de l'acétate d'éthyle. L'ensemble des phases organiques est lavé à l'eau, séché sur sulfate de sodium, filtré et évaporé sous vide. On purifie par recristallisation dans un mélange d'acétate d'éthyle et d'hexane.
On récupère 5,8 g de produit (rendement = 94,9%).
Point de fusion : 128-130°C.
Temps de rétention en HPLC (colonne Kromasil, 5 µm, 10x250 mm, acétonitrile - eau, 60%, 1 ml/min) = 6,4 et 6,6 min.

### 1.8. (R)-3-([1,1'-Biphényl]-4-yl)-2-[[hydroxy[1-[[(phénylméthoxy)carbonyl]amino]éthyl]phosphinyl]méthyl] propanoate d'éthyle.

On condense 6,4 g (12,5 mmol) de (*R*)-3-(4-bromophényl)-2-[[hydroxy[1-[[(phénylméthoxy)carbonyl]amino]éthyl] phosphinyl]méthyl]propanoate d'éthyle avec 1,54 g (12,6 mmol) d'acide phénylboronique en opérant dans 48 ml d'un mélange toluène / méthanol : 2 / 1, de tétrakis(triphénylphosphine) palladium et de 12,8 ml de carbonate de sodium 2M. Le mélange réactionnel est agité pendant 6 heures sous azote et à reflux. Après refroidissement, on ajoute 50 ml d'acétate d'éthyle et acidifie jusqu'à pH = 3 par une solution aqueuse d'acide chlorhydrique 2N. Après filtration sur célite®, rincé par de l'acétate d'éthyle, le solvant est évaporé sous pression réduite. On purifie par chromatographie sur gel de silice en éluanc avec un mélange dichlorométhane / méthanol / acide acétique : 9 / 1 / 0,4.
On récupère 5,3 g de produit (rendement = 83,5%).
Point de fusion :124-126°C.
Temps de rétention en HPLC (colonne Kromasil®, 5 µm,
10x250 mm, acétonitrile - eau, 60%, 1 ml/min) = 8,0 et 8,2 min.

### 1.9. (R)-3-([1,1'-Biphényl]-4-yl)-2-[[[1-[[(1,1-diméthyl_ éthoxy)carbonyl]amino] éthyl]hydroxyphosphinyl]méthyl] propanoate d'éthyle

A 10 ml d'une solution de 33% d'acide bromhydrique dans l'acide acétique, refroidi à 0°C, on ajoute 4,7 g (9,2 mmol) de (*R*)-3-([1,1'-biphényl]-4-yl)-2-[(hydroxy[1-[[(phénylméthoxy)carbonyl]amino]éthyl]phosphinyl]méthyl] propanoate d'éthyle. Le mélange est ensuite agité, à température ambiante, pendant 30 minutes. Le solvant est évaporé sous pression réduite. Le résidu est repris dans le toluène puis le cyclohexane et évaporé, sous pression réduite, à sec.
On récupère 4,2 g de bromhydrate de (*R*)-3-([1,1'-biphényl]-4-yl)-2-[[hydroxy[1-[[(phénylméthoxy)carbonyl]amino]éthyl] phosphinyl]méthyl]propanoate d'éthyle.
A une solution de 4,2 g (9,2 mmol) de ce sel dans 15 ml de diméthylformamide, on ajoute goutte à goutte, 5,1 ml de triéthylamine (36,7 mmol) et de 2 g (9,2 mmol) de di-*tert*-butyldicarbonate. A la fin de l'addition, le mélange est maintenu à une température voisine de 20°C pendant la nuit. Le solvant est concentré. On ajoute 15 ml d'eau et acidifie jusqu'à pH = 3 par une solution aqueuse d'acide chlorhydrique 2N. La phase aqueuse est extraite par de l'acétate d'éthyle. L'ensemble des phases organiques est lavé à l'eau, séché sur sulfate de sodium, filtré et évaporé sous vide. On purifie par chromatographie sur gel de silice en éluant par un mélange dichlorométhane / méthanol / acide acétique : 9 / 1 / 0,4.
On récupère 3,6 g de produit (rendement = 82%).
Point de fusion : 114-116°C.
Temps de rétention en HPLC (colonne Kromasil®, 5 µm,
10x250 mm, acétonitrile - eau, 60%, 1 ml/min) = 8,4 et 8,7 min.

### 1.10. Acide (R)-3-([1,1'-biphényl]-4-yl)-2-[[[1-[[(1,1-diméthyléthoxy)carbonyl]amino]éthyl]hydroxy_ phosphinyl]méthyl]propanoïque

A une solution de 3,6 g (7,6 mmol) de (*R*)-3-([1,1'-biphényl]-4-yl)-2-[[[1-[[(1,1-diméthyléthoxy)carbonyl]amino]éthyl] hydroxyphosphinyl]méthyl]propanoate d'éthyle dans 35 ml d'éthanol, on ajoute 60 ml d'hydroxyde de lithium 1N. Après 24 heures d'agitation, on acidifie le milieu avec de l'acide chlorhydrique 3N. On évapore ensuite l'éthanol et on extrait trois fois avec de l'acétate d'éthyle. L'ensemble des phases organiques est lavé à l'eau, séché sur sulfate de sodium et évaporé.
On récupère 3,35 g de produit (rendement = 99%).
Point de fusion : 110°C (décomposition).
Temps de rétention en HPLC (colonne Kromasil, 5 µm, 10x250 mm, acétonitrile - eau, 60%, 1 ml/min) = 5,0 et 5,1 min.

### 1.11. (R,S)-N-[2-([1,1'-Biphényl]-4-ylméthyl)-3-[[(1-[[(1,1-diméthyléthoxy)carbonyl]amino]éthyl]hydroxyphosphinyl] -1-oxopropyl]-L-alaninate de phénylméthyle (isomère A (R,S,S) et isomère B (R,R,S))

A 3,4 g (7/5 mmol) d'acide (*R*)-3-([1,1'-biphényl]-4-yl)-2-[[[1-[[(1,1-àiméthyléthoxy)carbonyl]amino]éthyl]hydroxy_ phosphinyl]méthyl]propanoïque, 1,4 g (7,5 mmol) de *L*-alaninate de phénylméthyle sous forme de chlorhydrate et 6,8 g (15,1 mmol) de benzotriazol-1-yl-oxytris(diméthylamino)-phosphonium hexafluorophosphate (BOP) dans 12 ml de diméthylformamide, on ajoute sous agitation à température voisine de 20°C, 13 ml de diisopropyléthylamine. Après 30 minutes de réaction à cette température, on évapore le diméthylformamide sous pression réduite, on ajoute 50 ml d'acétate d'éthyle et acidifie jusqu'à pH = 3 par une solution aqueuse d'acide chlorhydrique 2N. La phase aqueuse est extraite par de l'acétate d'éthyle. L'ensemble des phases organiques est lavé à l'eau, séché sur sulfate de sodium, filtré et évaporé sous vide. On purifie par chromatographie sur gel de silice en éluant avec un mélange dichlorométhane /méthanol / acide acétique : 9 / 1 / 0,3.
On récupère 1 g de l'isomère A (23,3%) et 1,1 g de l'isomère B (25,6%).
Temps de rétention en HPLC (Colonne Kromasil® 5 µm, 10x250 mm, acétonitrile - eau : 60%, 1 ml/min) = 14,1 et 15,5 min.

### 1.12. Ethanethioate de S-(2-hydroxyéthyle)

A une solution de 8,2 g (107,7 mmol) de l'acide thioacétique dans 43 ml de toluène, on ajoute, à 0°C, 16,4 g (107,7 mmol) de 1,8-diazabicyclo[5.4.0]undec-7-ène et 16,1 g (93,7 mmol) de 2-iodoéthanol. Le mélange est maintenu à une température voisine de 20 °C pendant 3 heures. On ajoute un mélange d'acétate d'éthyle et d'eau. La phase organique est lavée à l'eau, séchée sur sulfate de sodium, filtrée et évaporée sous vide. Le résidu est repris par de l'éther, filtré et évaporé sous pression réduite.
On récupère 7,98 g de produit (rendement = 71%).

### 1.13. (R,S)-N-[3-[[2-(Acéthylthio)éthoxy][1-[[(1,1-diméthyl_ éthoxy)carbonyl]amino]éthyl]phosphinyl]-2-([1,1'-biphényl]-4-ylméthyl)-1-oxopropyl]-L-alaninate de phénylméthyle

A une solution de 100 mg (0,17 mmol) de (*R*,*S*)-*N*-[2-([1,1'-biphényl]-4-ylméthyl)-3-[[(1-[[(1,1-diméthyléthoxy)carbonyl] amino]éthyl]hydroxyphosphinyl]-1-oxopropyl]-*L*-alaninate de phénylméthyle (isomère A), dans 10 ml de tétrahydrofurane anhydre, on ajoute 103 mg d'éthanethioate de *S*-(2-hydroxyéthyle) (0,87 mmol), 32 mg de diméthylaminopyridine (0,26 mmol) et 179 mg de dicyclohexylcarbamidine (0,87 mmol). Le mélange est maintenu à une température voisine de 20°C pendant 2 jours. Après filtration, le filtrat est évaporé sous pression réduite. Le résidu est repris dans de l'acétate d'éthyle, filtré et évaporé sous pression réduite. On purifie par chromatographie sur gel de silice en éluant avec un mélange dichlorométhane /méthanol : 40 / 3.
On récupère 78 mg de produit (rendement = 66,4%).
Point de fusion : 78-80°C.
Temps de rétention en HPLC (Colonne Kromasil® 5 µm, 10x250 mm, acétonitrile - eau : 70%, 1 ml/min) = 8,7 min.

### 1.14. Formiate de(R,S)-N-[3-[[2-(acétylthio)éthoxy] (1-aminoéthyl)phosphinyl]-2-([1,1'-biphényl]-4-ylméthyl)-1-oxopropyl]-L-alaninate de phénylméthyle

Une solution de 78 mg (0,12 mmol) de (*R*,*S*)-*N*-[3-[[2-(acéthylthio)éthoxy][1-[[(1,1-diméthyléthoxy)carbonyl] amino] éthyl]phosphinyl]-2-([1,1'-biphényl]-4-ylméthyl)-1-oxopropyl] -L-alaninate de phénylméthyle dans 1 ml d'acide formique est maintenue sous agitation à une température voisine de 20 °C pendant 4 heures. Le solvant est évaporé sous pression réduite à sec. Le résidu est purifié par chromatographie préparative (Kromasil C₈, 10 mm, 20x250 mm, acétonitrile /eau : 50 / 50, 15 ml/min).
On récupère 71,8 mg de produit (rendement = 82%).
Point de fusion : 108-110°C.
Temps de rétention en HPLC (colonne Kromasil®, 5µm, 10x250 mm, acétonitrile / eau : 50%, 1 ml/min) : 6,4 et 6,9 min.

### Exemple 2 : Formiate de (R,S)-N-[3-[[1-(acétyloxy)-2-méthylpropoxy](1-aminoéthyl)phosphinyl]-2-([1,1'-biphényl]-4-ylméthyl)-1-oxopropyl]-L-alaninate de phénylméthyle

### 2.1. Acétate de 1-bromo-2-méthylpropyle

A une solution de 13,6 g (0,1 mol) de bromure d'acétyle dans 60 ml de dichlorométhane anhydre, on ajoute 1 g de chlorure de zinc, et goutte à goutte, 9,1 ml (0,1 mol) d'isobutyraldéhyde, de manière à maintenir la température de la réaction en dessous de -5°C. Après addition, le mélange est maintenu à cette température pendant 2 heures. Il est ensuite filtré sur alumine basique, puis rincé avec 20 ml de dichlorométhane anhydre. Le solvant est évaporé sous pression réduite. Le résidu est repris dans de l'éther, lavé à l'eau, séché sur sulfate de sodium et filtré. Le solvant est évaporé sous vide.
On récupère 16,2 g de produit (rendement = 83%).

### 2.2. (R,S)-N-[3-([1-(Acétyloxy)-2-méthylpropoxy][1-[[(1,1-diméthyléthoxy) carbonyl] amino]éthyl]phosphinyl]-2-([1,1'-biphényl]-4-ylméthyl)-1-oxopropyl]-L-alaninate de phénylméthyle

A une solution de 118 mg (0,21 mmol) de (*R*,*S*)-*N*-[2-([1,1'-biphényl]-4-ylméthyl)-3-[[(1-[[(1,1-diméthyléthoxy) carbonyl]amino]éthyl]hydroxyphosphinyl]-1-oxopropyl]-*L*-alaninate de phénylméthyle (isomère A) dans un mélange de 1,4 ml de méthanol et de 0,14 ml d'eau, on ajoute 16,7% de carbonate de cesium jusqu'à pH = 8-9. Le solvant est évaporé sous pression réduite, à sec. Le solide sous forme de sel de cesium est dissout dans 1,4 ml de diméthylformamide. On ajoute 200 mg (1 mmol) d'acétate de 1-bromo-2-méthylpropyle. Le mélange est maintenu à une température voisine de 20°C pendant la nuit. On ajoute un mélange d'acétate d'éthyle et d'eau. La phase organique est lavée à l'eau, séchée sur sulfate de sodium, filtrée et évaporée sous vide. On récupère 59 mg de produit (rendement =41,7%).
Point de fusion : 76-78°C.
Temps de rétention en HPLC (Colonne Kromasil® 5 µm, 10x250 mm, acétonitrile - eau : 70%, 1 ml/min) = 11,5 ; 12,4 et 13,2 min.

### 2.3. Formiate de (R,S)-N-[3-[[1-(acétyloxy)-2-méthylpropoxy](1-aminoéthyl)phosphinyl]-2-(1,1'-biphényl]-4-ylméthyl)-1-oxopropyl]-L-alaninate de phénylméthyle

On procède selon les conditions opératoires décrites en 1.12. à partir de 58 mg (0,08 mmol) de (*R*,*S*)-*N*-[3-[[1-(acétyloxy)-2-méthylpropoxy][1-[[(1,1-diméthylethoxy)carbonyl]amino] éthyl]phosphinyl]-2-([1,1'-biphényl]-4-ylméthyl)-1-oxopropyl] -*L*-alaninate de phénylméthyle et de 0,7 ml d'acide formique. On récupère 32 mg de produit (rendement = 60%).
Point de fusion : 120-122°C.
Temps de rétention en HPLC (Colonne Kromasil® 5 µm, 10x250 mm, acétonitrile - eau : 50%, 1 ml/min) = 7,1 ; 8,3 et 9,6 min.

### Exemple 3 : Formiate de (R,S)-N-[3-[[(2-(acétylthio) éthoxy](aminophénylméthyl)phosphinyl]-2-([1,1'-biphényl]-4-ylméthyl)-1-oxopropyl]-L-alaninate de phénylméthyle

### 3.1. Phosphinate de α-phénylbenzèneméthanamine

A une solution de 33,1 g (0,5 mol) d'acide phosphoneux 100% dans 120 ml d'éthanol anhydre, refroidi à 0°C, sont ajoutés goutte à goutte 91,9 g (0,5 mol) de diphénylméthylamine en veillant à ce que la température ne dépasse pas 25°C. Il se forme après addition un précipité blanc auquel on ajoute 200 ml de d'écher diéthylique. Le précipité est filtré, rincé à l'éther diéthylique puis séché.
On récupère 119,4 g de produit (rendement = 95,6%).
Point de fusion : 176-177°C.

### 3.2. Acide [[(diphénylméthyl)amino]phénylméthyl]phosphinique

Une solution de 110 g (0,44 mol) de phosphinate de α-phénylbenzèneméthanamine dans 310 ml d'éthanol anhydre est porté au reflux. 89,5 ml (0,88 mol) de benzaldéhyde dans 102 ml d'éthanol anhydre sont ajoutés goutte à goutte. Il se forme un précipité blanc abondant. Après addition, on maintient le reflux pendant 2,5 heures. Après refroidissement, on ajoute 840 ml d'acétone. Le précipité obtenu est filtré et lavé avec le même solvant. On récupère 75,3 g de produit (rendement = 50,6%).
Point de fusion : 212°C.

### 3.3. Acide (aminophénylméthyl)phosphinique

Un mélange de 75 g (0,22 mol) de l'acide
[[(diphénylméthyl)amino]phénylméthyl]phosphinique et de 509 ml de l'acide bromhydrique aqueux à 48% est porté à reflux pendant 2 heures. Deux phases apparaissent. Le mélange est mis à sec par évaporation sous vide à chaud puis repris dans 94 ml d'eau. On lave trois fois la phase aqueuse à l'éther diéthylique. La phase aqueuse, à nouveau évaporée, conduit au bromhydrate de l'acide (aminophénylméthyl) phosphinique, qui est repris dans 340 ml d'éthanol absolu. On ajoute 108 ml d'oxyde de propylène, goutte à goutte, sous agitation, à 0°C. On observe la formation d'un précipité blanc abondant qui est filtré et lavé à l'éther diéthylique. On récupère 36 g de produit (rendement = 94,6%).
Point de fusion : 243°C.

### 3.4. Acide [[[(phénylméthoxy)carbonyl]amino]phénylméthyl] phosphinique

On procède selon les conditions opératoires décrites en 1.5. à partir de 10 g (58,5 mmol) d'acide (aminophénylméthyl) phosphinique et de 9,8 ml (68,6 mmol) de chloroformiate de phénylméthyle.
On récupère 17,78 g de produit (rendement = 99,7%).
Point de fusion : 143°C.

### 3.5. Acide (R)-[[[(phénylméthoxy)carbonyl]amino]phénylméthyl] phosphinique

On procède selon les conditions opératoires décrites en 1.6. à partir de 9,2 g (21,6 mmol) d'acide [[[(phénylméthoxy) carbonyl]amino]phénylméthyl]phosphinique. On récupère 3,6 g de produit (rendement = 77,8%).
Point de fusion : 160°C.
[α]²⁰_{D} = +2,8°(c=1, éthanol).

### 3.6. (R)-3-(4-Bromophényl)-2[[hydroxy[[[(phénylméthoxy) carbonyl]amino]méthyl]phosphinyl]méthyl]propanoate d'éthyle

On procède selon les conditions opératoires décrites en 1.7. à partir de 15,7 g (51,4 mmol) d'acide (*R*)-[[[(phénylméthoxy) carbonyl]amino]phénylméthyl]phosphinique et de 16,6 g (61,7 mmol) de 4-bromo-α-méthylènebenzènepropanoate d'éthyle. On récupère 25,8 g de produit (rendement = 87,4%).
Point de fusion : 120-122°C.
Temps de rétention en HPLC (Colonne Kromasil® 5 µm, 10x250 mm, acétonitrile - eau : 60%, 1 ml/min) = 6,5 et 6,6 min.

### 3.7. (R)-3-[1,1'-Biphényl]-4-yl-2[[hydroxy[[[(phénylméthoxy) carbonyl]amino]méthyl]phosphinyl]méthyl]propanoate d'éthyle

On procède selon les conditions opératoires décrites en 1.8. à partir de 25 g (43,6 mmol) de (*R*)-3-(4-bromophényl)-2[[hydroxy[[[(phénylméthoxy)carbonyl]amino)méthyl]phosphinyl] méthyl]propanoate d'éthyle et de 5,4 g (43,8 mmol) de l'acide phénylboronique.
On récupère 21,7 g de produit (rendement = 87,3%).
Point de fusion : 116-118°C.
Temps de rétention en HPLC (Colonne Kromasil® 5 µm, 10x250 mm, acétonitrile - eau : 60%, 1 ml/min) = 9,6 et 9,8 min.

### 3.8. (R)-3-[1,1'-Biphényl)-4-yl-2[[[[[(1,1-diméthyléthoxy carbonyl]amino]méthyl]hydroxyphosphinyl]méthyl]propanoate d'éthyle

On procède selon les conditions opératoires décrites en 1.9. à partir de 15 g (26,3 mmol) de (*R*)-3-[1,1'-biphényl]-4-yl-2 ([hydroxy[[[(phénylméthoxy)carbonyl]amino]méthyl]phosphinyl] méthyl]propanoate d'éthyle.
On récupère 11,2 g de produit (rendement = 79,5%).
Point de fusion : 110-112°C.
Temps de rétention en HPLC (Colonne Kromasil® 5 µm, 10x250 mm, acétonitrile - eau : 60%, 1 ml/min) = 12,0 min.

### 3.9. Acide (R)-3-[1,1'-Biphényl]-4-yl-2[[[[[(1,1-diméthyl_ éthoxy)carbonyl]amino]méthyl]hydroxyphosphinyl]méthyl] propanoïque

On procède selon les conditions opératoires décrites en 1.10. à partir de 11,2 g (20,9 mmol) de (*R*)3-[1,1'-biphényl] -4-yl-2[[[[[(1,1-diméthyléthoxy)carbonyl]amino]méthyl] hydroxyphosphinyl]méthyl]propanoate d'éthyle.
On récupère 10,5 g de produit (rendement = 99%).
Point de fusion : 120°C (décomposition).
Temps de rétention en HPLC (Colonne Kromasil® 5 µm, 10x250 mm, acétonitrile - eau : 60%, 1 ml/min) = 6,7 min.

### 3.10. (R,S)-N-[3-([1,1'-Biphényl]-4-yl)-2-[[[[[(1,1-diméthyl_ éthoxy)carbonyl]amino]phénylméthyl]hydroxyphosphinyl]méthyl]-1-oxopropyl]-L-alaninate de phénylméthyle

On procède selon les conditions opératoires décrites en 1.11. à partir de 5 g (9,84 mmol) d'acide (*R*)-3-[1,1'-biphényl]-4-yl-2[[[[[(1,1-diméthyléthoxy)carbonyl]amino]méthyl] hydroxyphosphinyl]méthyl]propanoïque et 1,8 g (9,9 mmol) de *L*-alaninate de phénylméthyle sous forme de chlorhydrate. On récupère 1,3 g (20,7%) d'isomère A et 1,8 g (28,7%) d'isomère B.
Point de fusion : 143-146°C.
Temps de rétention en HPLC (Colonne Kromasil® 5 µm, 10x25C mm, acétonicrile - eau : 60%, 1 ml/min) = 13,7 et 15,2 min.

### 3.11. (R,S]-N-[3-[[2-(Acétylthio)éthoxy][[[(1,1-diméthyl_ éthoxy)carbonyl]amino]phénylméthyl]hydroxyphosphinyl]-2-([1,1'-biphényl]-4-ylméthyl)-1-oxopropyl]-L-alaninate de phénylméthyle

On procède selon les conditions opératoires décrites en 1.13. à partir de 210 mg (0,33 mmol) de (*R*,*S*)*-N*-[3-([1,1'-biphényl] -4-yl)-2-[[[[[(1,1-diméthyléthoxy)carbonyl]amino]phényl_ méthyl]hydroxyphosphinyl]méthyl]-1-oxopropyl]-*L*-alaninate de phénylméthyle et 195 mg (1,65 mmol) d'éthanethioate de *S*-(2-hydroxyéthyle).
On récupère 159 mg de produit (rendement = 69,6%).
Point de fusion : 102-104°C.
Temps de rétention en HPLC (Colonne Kromasil® 5 µm, 10x250 mm, acétonitrile - eau : 70%, 1 ml/min) = 12,3 min.

### 3.12. Formiate de (R,S)-N-[3-[[(2-(acétylthio) éthoxy](aminophénylméthyl)phosphinyl]-2-([1,1'-biphényl]-4-ylméthyl)-1-oxopropyl]-L-alaninate de phénylméthyle

On procède selon les conditions opératoires décrites en 1.14. à partir de 132 mg (0,18 mmol) de (*R*,*S*)-*N*-[3-[[2-(acéthylthio) éthoxy][[[(1,1-diméthyléthoxy)carbonyl]amino] phénylméthyl]hydroxyphosphinyl]-2-([1,1'-biphényl]-4-ylméthyl)-1-oxopropyl]-L-alaninate de phénylméthyle et 1,4 ml d'acide formique.
On récupère 83 mg de produit (rendement = 67,9%).
Point de fusion : 110-112°C.
Temps de rétention en HPLC (Colonne Kromasil® 5 µm, 10x250 mm, acétonitrile - eau : 50%, 1 ml/min) = 9,2 et 10,2 min.

### Exemple 4 : Formiate de (R,S)-N-[3-[[1-(acétyloxy)-2-méthyl propoxy](aminophénylméthyl)phosphinyl]-2-([1,1'-biphényl)-4-ylméthyl)-1-oxopropyl]-L-alaninate de phénylméthyle

### 4.1. (R,S)-N-[3-[[1-(Acétyloxy)-2-méthylpropoxy][[[(1,1-diméthyléthoxy)carbonyl]amino]phénylméthyl]phosphinyl]-2-([1,1'-biphényl]-4-ylméthyl)-1-oxopropyl]-L-alaninate de phénylméthyle

On procède selon les conditions opératoires décrites en 1.13. à partir de 100 mg (0,16 mmol) de (*R*,*S*)-*N*-[3-([1,1'-biphényl]-4-yl)-2-[[[[[(1,1-diméthyléthoxy)carbonyl]amino] phénylméthyl]hydroxyphosphinyl]méthyl]-1-oxopropyl]-*L*-alaninate de phénylméthyle et de 153 mg (0,78 mmol) d'acétate de 1-bromo-2-méthylpropyle.
On récupère 36 mg de produit (rendement = 31%).
Point de fusion : 80-82°C.
Temps de rétention en HPLC (Colonne Kromasil 5 µm, 10x250 mm, acétonitrile - eau : 70%, 1 ml/min) = 18,3 ; 19,6 et 21,5 min.

### 4.2. Formiate de (R,S)-N-[3-[[1-(acétyloxy)-2-méthyl_ propoxy](aminophénylméthyl)phosphinyl]-2-([1,1'-biphényl)-4-ylméthyl)-1-oxopropyl]-L-alaninate de phénylméthyle

On procède selon les conditions opératoires décrites en 1.14. à partir de 35 mg (0,047 mmol) de (*R*,*S*)*-N-*[3-[[1*-*(acétyloxy)-2-méthylpropoxy][[[(1,1-diméthyléthoxy)carbonyl]amino]phényl_ méthyl]phosphinyl]-2-([1,1'-biphényl]-4-ylméthyl)-1-oxo_ propyl]-*L*-alaninate de phénylméthyle et 0,4 ml d'acide formique.
On récupère 19,5 mg de produit (rendement = 60%).
Point de fusion : 132-134°C.
Temps de rétention en HPLC (Colonne Kromasil 5 µm, 10x250 mm, acétonitrile - eau : 50%, 1 ml/min) = 8,5 ; 12,2 et 15,0 min.

Pour synthétiser les composés de formule (I) d'une autre configuration on procède de la même façon, à partir des intermédiaires possédant la configuration souhaitée.

Le tableau suivant illustre quelques composés de l'invention, ainsi que leurs caractéristiques physiques.

Lorsque n est égal à un dans ce tableau, R₇ représente un atome d'hydrogène.

De plus, le phosphore est un centre chiral et le groupe R₄ peut également comporter un ou plusieurs centres chiraux. De ce fait, lorsque les centres chiraux du squelette sont résolus, comme dans les exemples, on obtient un mélange d'énantionères ou de diastéréoisomères. Par convention, on les appelle 1, 2, 3 etc... par ordre de temps de rétention croissant dans les conditions de chromatographie décrites dans le texte.

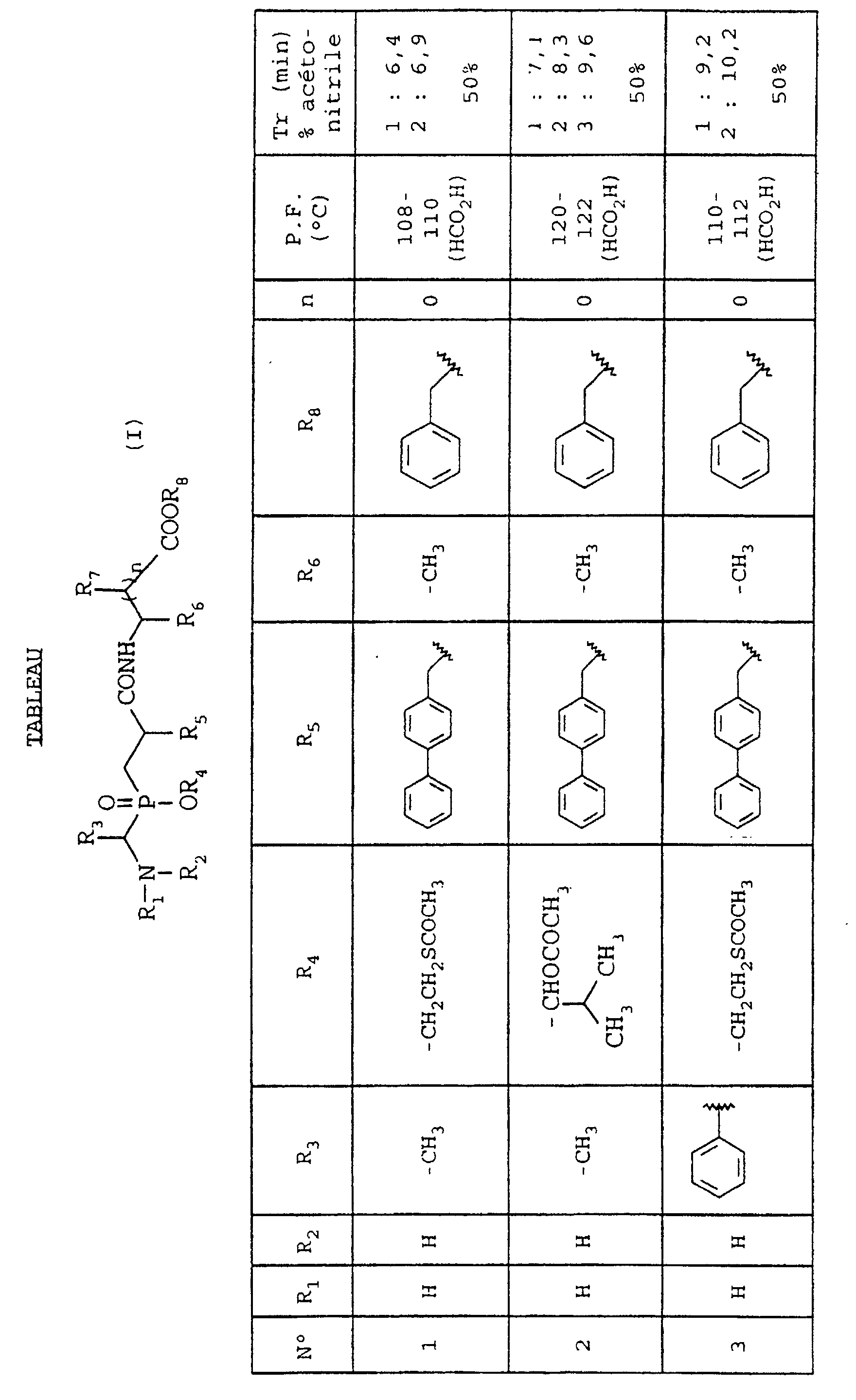

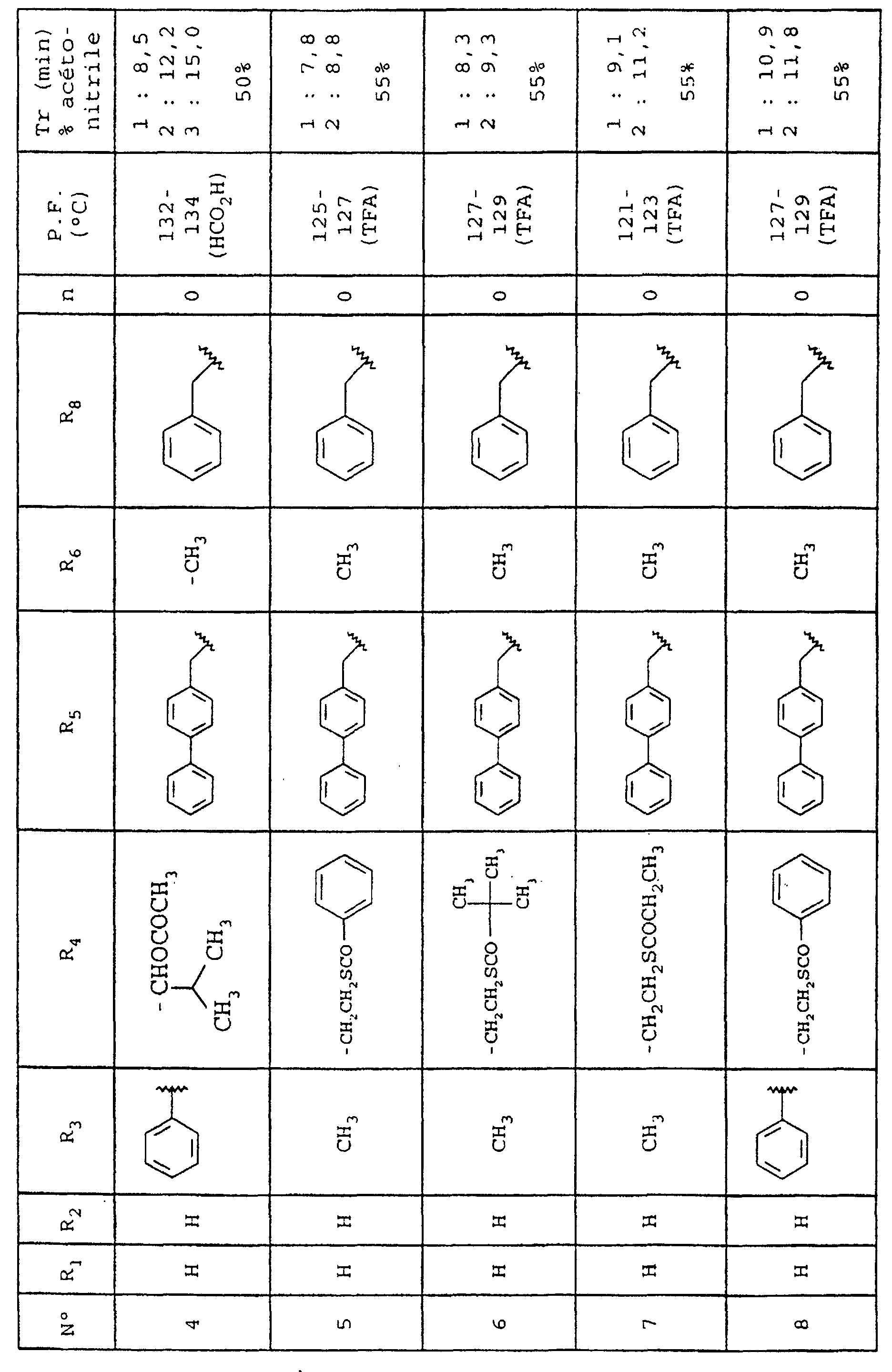

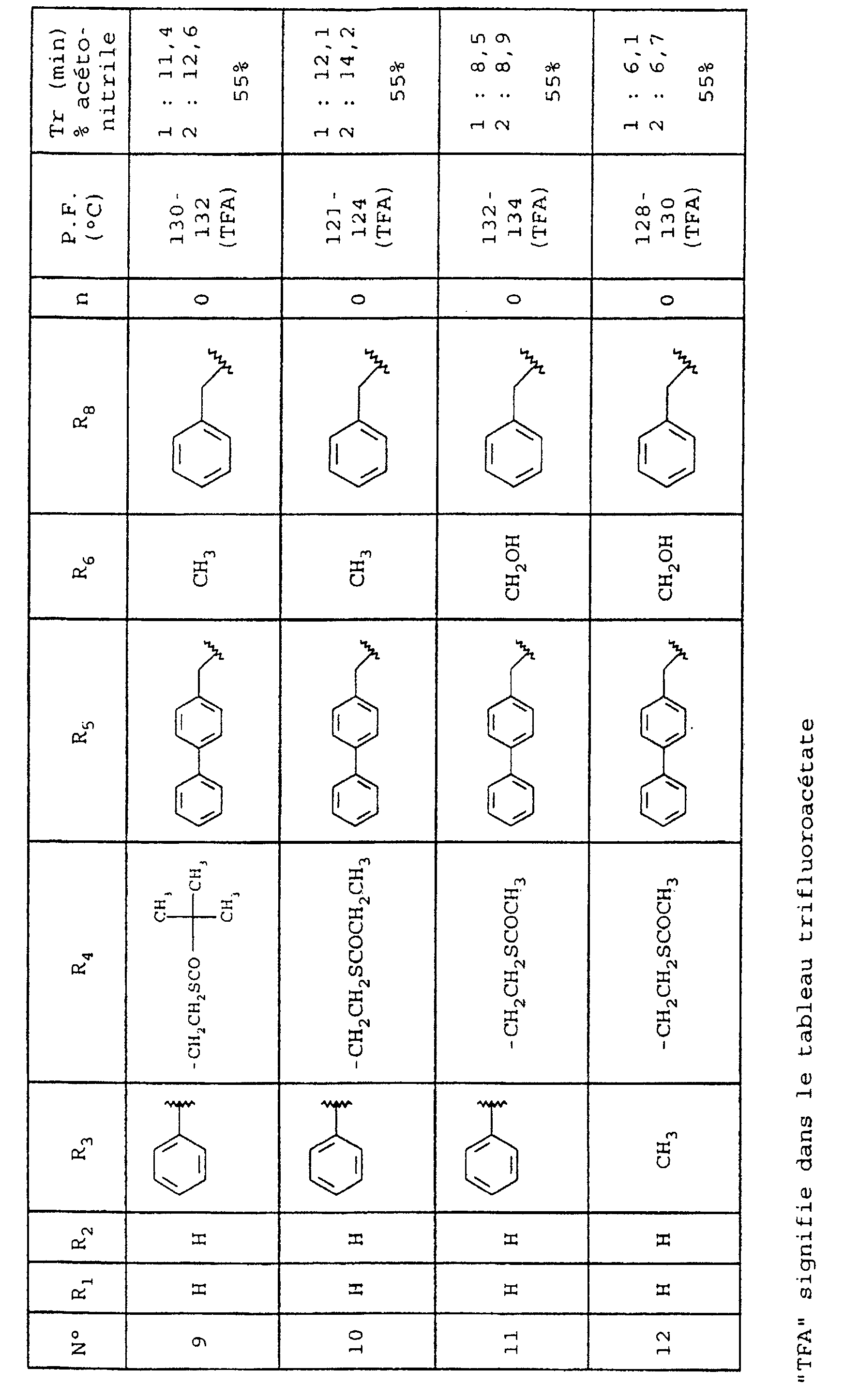

Les composés de l'invention ont fait l'objet d'essais enzymologiques permettant de déterminer leur pouvoir inhibiteur de l'EPN et de l'APN.

### Mesure du pouvoir inhibiteur sur l'endopeptidase neutre (EPN)

Le pouvoir inhibiteur est déterminé sur de l'endopeptidase neutre purifiée de rein de lapin en suivant le protocole décrit dans la littérature (Llorens et al., *Neurochem*., **39,** 1081, 1982). Après une incubation de 15 minutes à 25 °C, un aliquot de protéines est incubé pendant 20 minutes à 37°C en présence de 20 nmoles de (³H)D-Ala²-Leu⁵-enképhaline et du composé à tester en solution dans le tampon Tris HCl (pH = 7,4).
On arrête la réaction par addition d'acide chlorhydrique 0,2 N. Le métabolite tritié (³H)-Tyr-D-Ala-Gly est séparé de la D-Ala²-Leu⁵-enképhaline par chromatographie sur colonne de Porapak et la quantité de métabolite formée mesurée à l'aide d'un compteur à scintillation liquide.

L'activité des différents composés de l'invention, exprimée en concentrations inhibitrices 50 (CI₅₀) varie de 10⁻⁶ à 10⁻⁹ M.

### Pouvoir inhibiteur sur l'aminopeptidase N (APN)

Le pouvoir inhibiteur est mesuré sur l'aminopeptidase purifiée du rein de porc (Boehringer, France). Après une préincubation de 15 minutes à 25°C, un aliquot de protéines est incubé pendant 20 minutes à 25 °C en présence de 20 nmoles de (³H)-Leu-enképhaline et du composé à tester en solution dans un tampon tris Hcl (pH = 7,4).
On arrête la réaction par addition d'acide chlorhydrique 0,5 N. Le métabolite formé (³H)Tyr est séparé par chromatographie sur colonne de Porapak et la quantité de métabolite formée, mesurée à l'aide d'un compteur à scintillation liquide.

L'activité des différents composés de l'invention, exprimée en concentrations inhibitrices 50 (CI₅₀) varie de 10⁻⁶ à 10⁻⁹ M.

Les composés de l'invention ont également fait l'objet d'essais pharmacologiques permettant de mesurer leur activité analgésique.

### Test de la plaque chaude chez la souris

Le test est effectué 15 minutes après administration par voie intracérébroventriculaire de doses croissantes des composés de l'invention chez la souris.
On mesure deux paramètres : le temps de latence du saut et la latence du léchage.
Les résultats sont exprimés en DE₅₀, c'est-à-dire en doses donnant la moitié de la réponse maximale. L'activité analgésique des différents composés de l'invention se situe entre 1 et 100 µg/kg. L'activité des composés de l'invention les plus actifs se. situe entre 1 et 20 µg/kg.

Un autre protocole consiste à étudier l'effet des composés après administration par voie intraveineuse à des temps de 30, 60, 90, 120, 150 et 180 min, à la dose de 50 mg/kg.
Les paramètres mesurés sont le temps de latence du saut et le temps de latence du léchage.
L'activité analgésique des composés est exprimée en pourcentage de temps de latence par rapport au temps de latence maximal de 240 secondes.
L'activité des composés les plus actifs se situe entre 30 et 70%.

Un troisième protocole consiste à étudier l'effet antinociceptif des composés après administration par voie intrapéritonéale à des temps de 30, 60, 90, 120, 150 et 180 min après l'administration. Les composés sont administrés à une dose unique de 50 mg/kg.
L'activité analgésique est exprimée en pourcentage du temps de latence par rapport au temps de latence maximun (240 secondes).
L'activité des composés les plus actifs se situe entre 30 et 70% d'analgésie.

Les composés de l'invention présentent une activité mixte EPN/APN in vitro et une activité analgésique in vivo ; ces résultats montrent que les composés de l'invention peuvent être utilisés comme analgésiques.

Les composés selon l'invention peuvent aussi être mis en oeuvre pour la préparation de médicaments destinés au traitement des états dépressifs de toute nature, des troubles du sommeil, des troubles de l'anxiété, des troubles cognitifs et de la vigilance, et des troubles d'ordre périphériques (diarrhée, toux, hypertension, inflammation...).

L'invention a également pour objet des compositions pharmaceutiques comprenant à titre de principe actif l'un au moins des composés de formule (I) ou leurs sels d'addition en association avec tout excipient approprié.

Les composés de l'invention peuvent. être présentés, en association avec des excipients, sous forme de compositions formulées en vue de l'administration par voie entérale ou parentérale, par exemple sous forme de comprimés, de pilules, de granulés, de poudres, de dragées, de capsules, de solutions, de suspensions, de solutions injectables, d'élixirs ou de sirops.
Les solutions des sels des composés de l'invention sont particulièrement utiles pour l'administration par injection intramusculaire ou sous-cutanée.

Les composés de l'invention sont administrés à une dose journalière comprise entre 0,01 et 100 mg/kg, de préférence entre 0,1 et 10 mg/kg.

### annexe 1

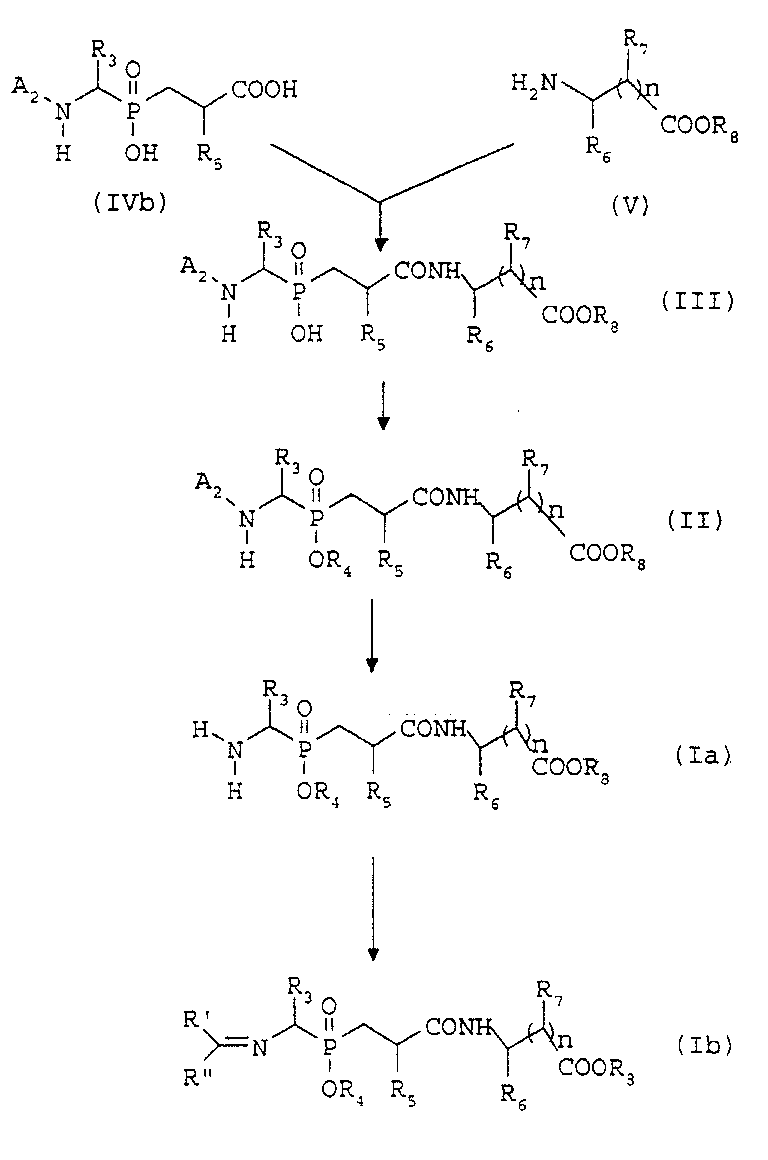

### annexe 2

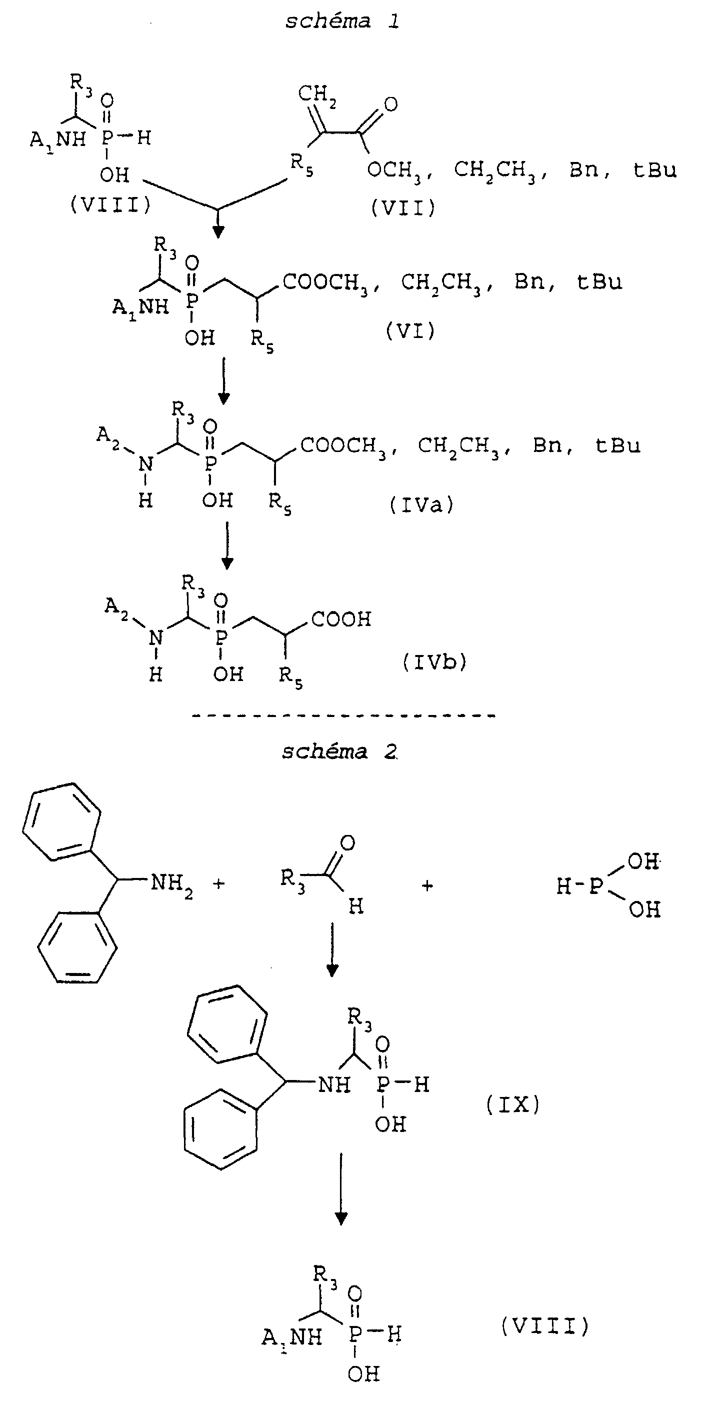

### annexe 3

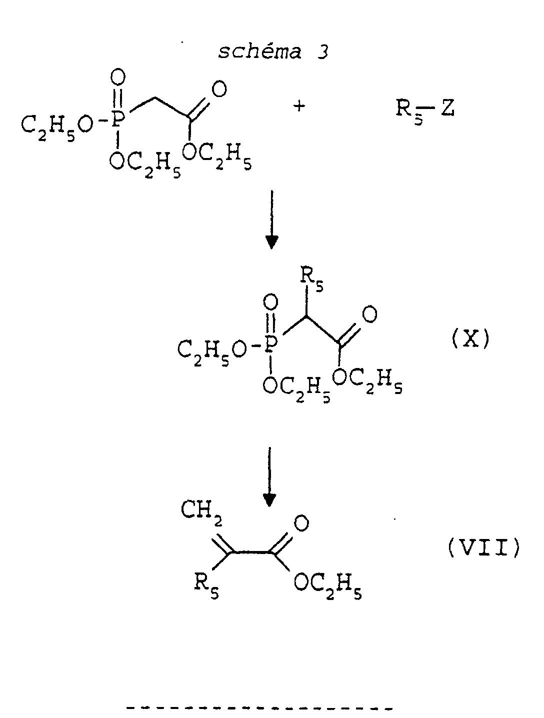

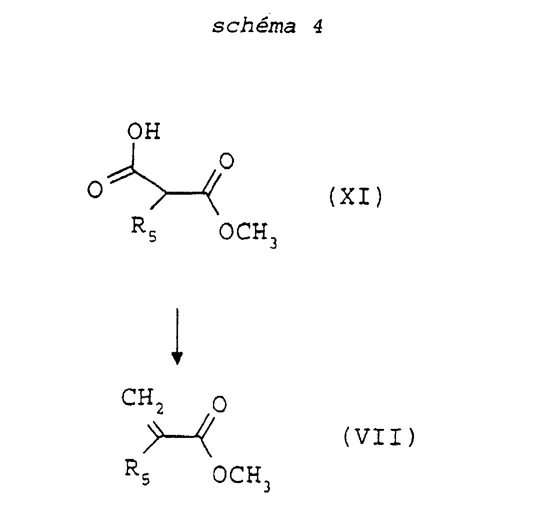

## Revendications

1. Composé de formule (I) 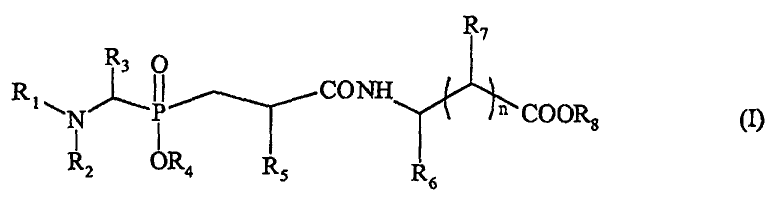 dans laquelle
• R₁ et R₂ représentent chacun un atome d'hydrogène ou bien R₁ et R₂, pris ensemble, forment un groupe insaturé de formule R'(R")C=, dans laquelle,
• R' représente un groupe phényle monosubstitué en position 2 par un groupe hydroxy ou bien un groupe phényle disubstitué, en position 2, par un groupe hydroxy et, en position 4 ou 5, soit par un atome d'halogène soit par un groupe nitro, soit par un groupe hydroxy, soit par un groupe alcoxy -OR₉,
• R" représente un groupe phényle, un groupe phényle substitué par 1 à 5 atomes d'halogène ou un groupe hétérocyclique aromatique,
• R₃ représente
• un atome d'hydrogène,
• un groupe alkyle ou un groupe alkényle de 1 à 6 atomes de carbone, ces deux derniers groupes pouvant être substitués par :
• un groupe hydroxy ou un groupe alcoxy -OR₉,
• un groupe phényle ou un groupe benzyle,
• un groupe sulfanyle, un groupe alkylsulfanyle -SR₉ ou un groupe alkylsulfanyle oxydé sur l'atome de soufre -S(O)R₉,
• un groupe amino, un groupe -NHR₉ ou -NR₉R₁₀, éventuellement oxydé sur l'atome d'azote ou,
• un groupe guanidino H₂N-C(=NH)-NH-,
• un groupe cycloalkyle ou cycloalkylméthyle,
• un groupe phényle, un groupe benzyle, pouvant être substitués sur le groupe phényle par 1 ou 2 des substituants suivants :
• un atome d'halogène,
• un groupe hydroxy, un groupe alcoxy -OR₉,
• un groupe alkylsulfanyle -SR₉ ou un groupe alkylsulfanyle oxydé sur l'atome de soufre,
• un groupe amino ou un groupe -NHR₉ ou un groupe -NR₉R₁₀ éventuellement oxydé sur l'atome d'azote,
• un groupe nitro,
• un groupe phényle,
• un groupe alkyle de 1 à 4 atomes de carbone,
• un groupe méthyle substitué par un groupe hétérocyclique aromatique ou saturé, les hétéroatomes pouvant être oxydés sous forme de N-oxyde ou de S-oxyde,
• R₄ représente
• un groupe -CH(X)-O-C(O)-Y, où X et Y représentent, indépendamment l'un de l'autre, un groupe R₉ ou un groupe phényle,
• un groupe -CH₂CH₂-S-C(O)-W, où W représente un groupe R₉ ou un groupe phényle,
• R₅ représente
• un atome d'hydrogène,
• un groupe alkyle ou un groupe alkényle de 1 à 6 atomes de carbone, ces deux derniers groupes pouvant être substitués par :
• un groupe hydroxy ou un groupe alcoxy -OR₉,
• un groupe phényle ou un groupe benzyle,
• un groupe sulfanyle, un groupe alkylsulfanyle -SR₉ ou un groupe alkylsulfanyle oxydé sur l'atome de soufre -S(O)R₉,
• un groupe amino, un groupe -NHR₉ ou -NR₉R₁₀, éventuellement oxydé sur l'atome d'azote ou,
• un groupe guanidino H₂N-C(=NH)-NH-,
• un groupe cycloalkyle ou cycloalkylméthyle,
• un groupe phényle, un groupe benzyle, pouvant être substitués sur le groupe phényle par 1 ou 2 des substituants suivants :
• un atome d'halogène,
• un groupe hydroxy, un groupe alcoxy -OR₉,
• un groupe alkylsulfanyle -SR₉ ou un groupe alkylsulfanyle oxydé sur l'atome de soufre,
• un groupe amino ou un groupe -NHR₉ ou -NR₉R₁₀ éventuellement oxydé sur l'atome d'azote,
• un groupe nitro,
• un groupe phényle,
• un groupe alkyle de 1 à 4 atomes de carbone,
• un groupe méthyle substitué par un groupe hétérocyclique, les hétéroatomes pouvant être oxydés sous forme de N-oxyde ou de S-oxyde,
• R₆ et R₇ représentent indépendamment l'un de l'autre
• un atome d'hydrogène
• un groupe alkyle ou alkényle de 1 à 6 atomes de carbone, pouvant être substitué par :
* un groupe hydroxy ou un groupe alcoxy -OR₉,
* un groupe sulfanyle, un groupe alkylsulfanyle -SR₉ ou un groupe alkylsulfanyle oxydé sur l'atome de soufre -S(O)R₉,
* un groupe amino ou un groupe alkylamino -NHR₉,
* un groupe guanidino H₂N-C(=NH)-NH- ou,
* un groupe carboxy ou un groupe alkyloxycarbonyle -COOR₉,
• un groupe phényle, un groupe benzyle, pouvant être substitués sur le groupe phényle par 1 ou 2 des substituants suivants :
* un atome d'halogène,
* un groupe phényle,
* un groupe hydroxy ou un groupe alcoxy -OR₉,
* un groupe alkylsulfanyle -SR₉ ou un groupe alkylsulfanyle oxydé sur l'atome de soufre -S(O)R₉,
• R₆ et R₇ représentent ensemble un cycle saturé ou insaturé à 5 ou 6 chaînons, comprenant 1 ou 2 hétéroatomes pris parmi l'oxygène, le soufre et l'azote,
• R₈ représente
• un groupe alkyle ou alkényle de 1 à 6 atomes de carbone,
• un groupe phényle, un groupe benzyle,
• R₉ et R₁₀ représentent chacun un groupe alkyle de 1 à 6 atomes de carbone, et
• n est égal à 0 ou 1,
sous forme d'isomères optiques, à savoir sous forme d'énantiomères et de diastéréoisomères et de mélanges de ces différentes formes, y compris de mélanges racémiques ainsi que leurs sels d'addition aux acides pharmacologiquement acceptables, pour lesquels R₁ et R₂ sont des atomes d'hydrogène.

2. Composé selon la revendication 1 **caractérisé en ce que**
• R₁ et R₂ représentent des atomes d'hydrogène,
• n est égal à 0,
• R₃ représente
• un atome d'hydrogène,
• un groupe alkyle de 1 à 6 atomes de carbone, pouvant être substitué par un groupe hydroxy, un groupe alcoxy -OR₉, un groupe sulfanyle ou un groupe alkylsulfanyle -SR₉,
• un groupe phényle, un groupe benzyle, pouvant être substitués sur le groupe phényle par un atome d'halogène, un groupe alkyle de 1 à 4 atomes de carbone, un groupe hydroxy, un groupe alcoxy -OR₉ ou un groupe alkylsulfanyle -SR₉,
• R₄ représente
• un groupe -CH(X)-O-C(O)-Y, où X et Y représentent, indépendamment l'un de l'autre, un groupe R₉ ou un groupe phényle,
• un groupe -CH₂CH₂-S-C(O)-W, où W représente un groupe R₉ ou un groupe phényle,
• R₅ représente
• un groupe phényle, un groupe benzyle, pouvant être substitués sur le groupe phényle par un atome d'halogène, un groupe alkyle de 1 à 4 atomes de carbone, un groupe hydroxy, un groupe alcoxy -OR₉ ou un groupe alkylsulfanyle -SR₉,
• un groupe biphénylméthyle,
• R₆ représente un groupe alkyle de 1 à 6 atomes de carbone, pouvant être substitué par un groupe hydroxy, un groupe alcoxy -OR₉, un groupe sulfanyle ou un groupe alkylsulfanyle -SR₉,
• R₈ représente
• un groupe alkyle de 1 à 6 atomes de carbone,
• un groupe phényle, un groupe benzyle.

3. Procédé de préparation des composés de formule (Ia) 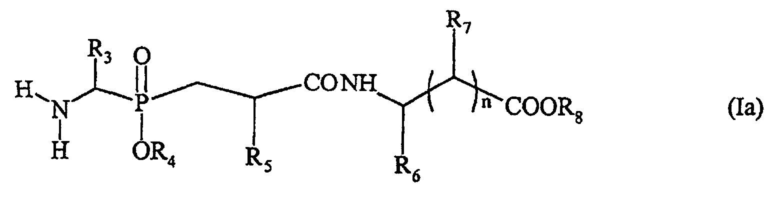 dans laquelle R₃, R₄, R₅, R₆, R₇, R₈ et n sont tels que définis dans la revendication 1, qui consiste :
- à traiter un composé de formule (IVb) 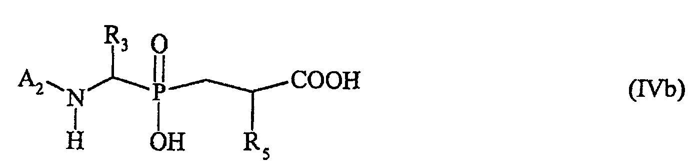 avec un composé de formule (V), lequel composé peut être utilisé sous forme de sel, tel que le sel de l'acide p-toluènesulfonique, 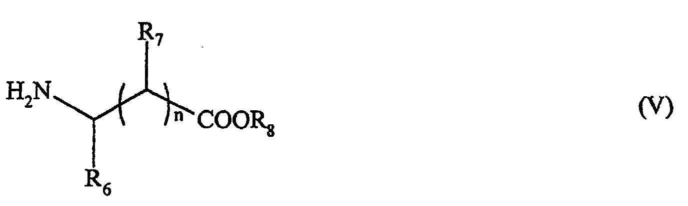 dans lesquelles
R₃, R₅, R₆, R₇, R₈ et n sont tels que définis dans la revendication 1, et A₂ représente un groupe tert-butoxycarbonyle ou un groupe fluorénylméthoxycarbonyle en présence de benzotriazol-1-yloxy-tris(diméthylamino)phosphonium hexafluorophosphate (BOP) ou bien de 1-éthyl-3-(3-diméthylaminopropyl)carbodiimide méthiodide (EDC), dans un solvant organique tel que le diméthylformamide, pour obtenir un composé de formule (III) 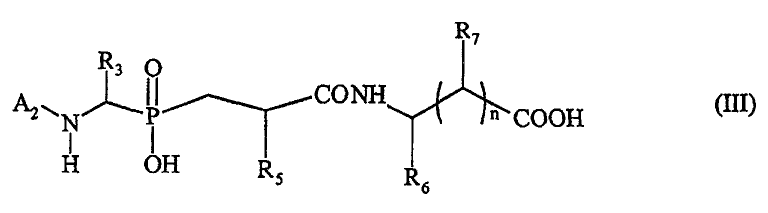 lequel est traité, dans un solvant organique tel que le tétrahydrofurane, par un alcool de formule R₄OH, dans laquelle R₄ est tel que défini dans la revendication 1, en présence de N,N'-dicyclohexylcarbodiimide et de 4-diméthylaminopyridine pour obtenir le composé de formule (II) 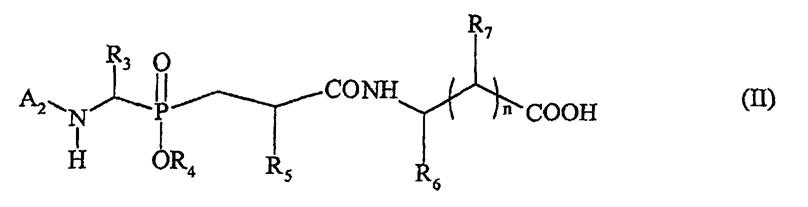 puis à déprotéger la fonction amine pour obtenir un composé de formule (Ia), l'étape de déprotection étant effectuée dans un milieu acide doux, tel que l'acide formique, si le groupe A₂ représente un groupe tert-butoxycarbonyle, ou dans un milieu basique, tel que la pipéridine, si le groupe A₂ représente un groupe fluorénylméthoxycarbonyle, ou bien,
- à transformer un composé de formule (III) tel qu'obtenu précédemment en sel de césium par action de carbonate de césium, et à traiter ce sel par un dérivé halogéné R₄Z, où Z représente un atome d'halogène tel que le brome, le chlore ou l'iode et R₄ est tel que défini précédemment, pour obtenir le composé de formule (II), puis à déprotéger la fonction amine pour obtenir un composé de formule (Ia), l'étape de déprotection étant effectuée dans un milieu acide doux, tel que l'acide formique, si le groupe A₂ représente un groupe tert-butoxycarbonyle, ou dans un milieu basique, tel que la pipéridine, si le groupe A₂ représente un groupe fluorénylméthoxycarbonyle.

4. Procédé de préparation des composés de formule (Ib) 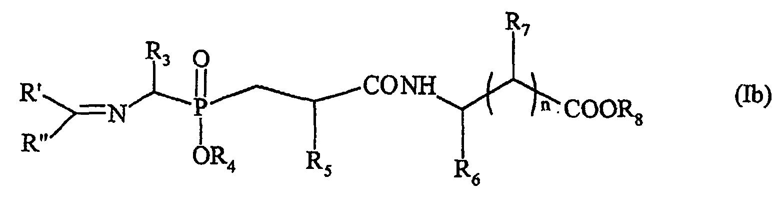 dans laquelle R', R", R₃, R₄, R₅, R₆, R₇, R₈ et n sont tels que définis dans la revendication 1, qui consiste à condenser une cétone R'(R")C=O sur un composé de formule (Ia), tel que préparé dans la revendication 3, ces cétones R'(R")C=O étant obtenues par réarrangement de Fries des esters correspondants R"CO₂R'.

5. Médicament **caractérisé en ce qu'**il est constitué par un composé de formule (I) selon la revendication 1 ou 2, lequel médicament est destiné au traitement des états dépressifs de toute nature, des troubles du sommeil, des troubles de l'anxiété, des troubles cognitifs et de la vigilance, et des troubles d'ordre périphérique tels que la diarrhée, la toux et l'inflammation.

6. Composition pharmaceutique **caractérisée en ce qu'**elle contient un composé selon la revendication 1 ou 2, en association avec tout excipient approprié.

## Patentansprüche

1. Verbindung der Formel (I) 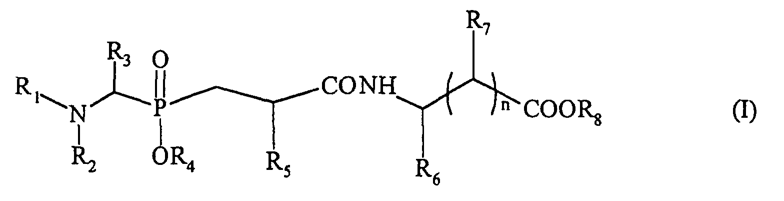 in welcher
• R₁ und R₂ jeweils ein Wasserstoffatom darstellen oder R₁ und R₁ und R₂ zusammengenommen eine ungesättigte Gruppe der Formel R'(R")C= bilden, in welcher
• R' eine durch eine Hydroxylgruppe an. Position 2 monosubstituierte Phenylgruppe darstellt oder eine an Position 2 durch eine Hydroxylgruppe und an Position 4 oder 5 entweder durch ein Halogenatom, oder durch eine Nitrogruppe oder durch eine Hydroxylgruppe oder durch eine Alkoxygruppe-OR₉ disubstituierte Phenylgruppe darstellt,
• R" eine Phenylgruppe, eine durch 1 bis 5 Halogenatome substituierte Phenylgruppe oder eine aromatische heterocyclische Gruppe darstellt,
• R₃ darstellt
• ein Wasserstoffatom,
• eine Alkylgruppe oder eine Alkenylgruppe mit 1 bis 6 Kohlenstoffatomen; wobei die beiden letzteren Gruppe substituiert sein können durch:
• eine Hydroxylgruppe oder eine Alkoxygruppe -OR₉,
• eine Phenylgruppe oder eine Benzylgruppe,
• eine Sulfanylgruppe, eine Alkylsulfanylgruppe -SR₉ oder eine über das Schwefelatom oxidierte Alkylsulfanylgruppe -S(O)R₉,
• eine Aminogruppe, eine Gruppe -NHR₉ oder -NR₉R₁₀, die gegebenenfalls über das Stickstoffatom oxidiert ist, oder
• eine Guanidinogruppe H₂N-C(=NH)-NH-,
• eine Cycloalkyl- oder Cycloalkylmethylgruppe,
• eine Phenylgruppe, eine Benzylgruppe, die über die Phenylgruppe durch ein oder zwei der folgenden Substituenten substituiert sein können:
• ein Halogenatom,
• eine Hydroxylgruppe, eine Alkoxygruppe-OR₉,
• eine Alkylsulfanylgruppe-SR₉ oder eine über das Schwefelatom oxidierte Alkylsulfanylgruppe,
• eine Aminogruppe oder eine Gruppe -NHR₉ oder eine Gruppe -NR₉R₁₀, die gegebenenfalls über das Stickstoffatom oxidiert ist,
• eine Nitrogruppe,
• eine Phenylgruppe,
• eine Alkylgruppe mit 1 bis 4 Kohlenstoffatomen,
• eine durch eine aromatische oder gesättigte heterocyclische Gruppe substituierte Methylgruppe, wobei die Heteroatome in der Form des N-Oxids oder S-Oxids oxidiert sein können,
R₄ darstellt
• eine Gruppe -CH(X)-O-C(O)-Y, wobei X oder Y unabhängig voneinander eine Gruppe R₉ oder eine Phenylgruppe darstellen,
• eine Gruppe -CH₂CH₂-S-C(O)-W, wobei W eine Gruppe R₉ oder eine Phenylgruppe darstellt,
R₅ darstellt
• ein Wasserstoffatom,
• eine Alkylgruppe oder eine Alkenylgruppe mit 1 bis 6 Kohlenstoffatomen, wobei die beiden letzteren Gruppen substituiert sein können durch:
• eine Hydroxylgruppe oder eine Alkoxygruppe -OR₉,
• eine Phenylgruppe oder eine Benzylgruppe,
• eine Sulfanylgruppe, eine Alkylsulfanylgruppe -SR₉ oder eine über das Schwefelatom oxidierte Alkylsulfanylgruppe -S(O)R₉,
• eine Aminogruppe, eine Gruppe -NHR₉ oder -NR₉R₁₀, die gegebenenfalls über das Stickstoffatom oxidiert ist, oder
• eine Guanidinogruppe H₂N-C(=NH)-NH-,
• eine Cycloalkyl- oder Cycloalkylmethylgruppe,
• eine Phenylgruppe, eine Benzylgruppe, die über die Phenylgruppe durch 1 oder 2 der folgenden Substituenten substituiert sein können:
• ein Halogenatom,
• eine Hydroxylgruppe, eine Alkoxygruppe -OR₉,
• eine Alkylsulfanylgruppe -SR₉ oder eine über das Schwefelatom oxidierte Alkylsulfanylgruppe,
• eine Aminogruppe oder eine Gruppe -NHR₉ oder -NR₉R₁₀, die gegebenenfalls über das Stickstoffatom oxidiert sind,
• eine Nitrogruppe,
• eine Phenylgruppe,
• eine Alkylgruppe mit 1 bis 4 Kohlenstoffatomen,
• eine Methylgruppe, die durch eine heterocyclische Gruppe substituiert ist, wobei die Heteroatome in der Form von N-Oxid oder von S-Oxid oxidiert sein können,
• R₆ und R₇ unabhängig voneinander darstellen
• ein Wasserstoffatom,
• eine Alkyl- oder Alkenylgruppe mit 1 bis 6 Kohlenstoffatomen, die substituiert sein können durch
* eine Hydroxylgruppe oder eine Alkoxygruppe -OR₉,
* eine Sulfanylgruppe, eine Alkylsulfanylgruppe -SR₉ oder eine über das Schwefelatom oxidierte Alkylsulfanylgruppe -S(O)R₉,
* eine Aminogruppe oder eine Alkylaminogruppe -NHR₉,
* eine Guanidinogruppe H₂N-C(=NH)-NH-, oder
* eine Carboxylgruppe oder eine Alkoxycarbonylgruppe -COOR₉,
• eine Phenylgruppe, eine Benzylgruppe, die über die Phenylgruppe durch 1 oder 2 der folgenden Substituenten substituiert sein können:
* ein Halogenatom,
* eine Phenylgruppe,
* eine Hydroxylgruppe oder eine Alkoxygruppe -OR₉,
* eine Alkylsulfanylgruppe-SR₉ oder eine über das Schwefelatom oxidierte Alkylsulfanylgruppe -S(O)R₉,
• R₆ und R₇ zusammen einen gesättigten oder ungesättigten Cyclus mit 5 oder 6 Kettengliedern darstellen, die ein oder zwei Heteroatome ausgewählt aus Sauerstoff, Schwefel und Stickstoff umfassen,
• R₈ darstellt
• eine Alkylgruppe oder Alkenylgruppe mit 1 bis 6 Kohlenstoffatomen,
• eine Phenylgruppe, eine Benzylgruppe,
• R₉ und R₁₀ jeweils eine Alkylgruppe mit 1 bis 6 Kohlenstoffatomen darstellen, und
• n gleich 0 oder 1 ist,
in der Form von optischen Isomeren, d.h. in der Form von Enantiomeren und von Diastomeren und von Mischungen der verschiedenen Formen, wobei racemische Mischungen darunter umfasst sind, genau so wie deren Additionssalze mit pharmakologisch akzeptablen Säuren, für welche R₁ und R₂ Wasserstoffatome sind.

2. Verbindung gemäß Anspruch 1, **dadurch gekennzeichnet, dass**
• R₁ und R₂ Wasserstoffatome darstellen,
• n gleich 0 ist,
• R₃ darstellt
• ein Wasserstoffatom,
• eine Alkylgruppe mit 1 bis 6 Kohlenstoffatomen, die durch eine Hydroxylgruppe, eine Alkoxygruppe -OR₉, eine Sulfanylgruppe oder eine Alkylsulfanylgruppe -SR₉ substituiert sein kann,
• eine Phenylgruppe, eine Benzylgruppe, die durch ein Halogenatom, eine Alkylgruppe mit 1 bis 4 Kohlenstoffatomen, eine Hydroxylgruppe, eine Alkoxygruppe -OR₉ oder eine Alkylsulfanylgruppe -SR₉ über die Phenylgruppe substituiert sein können,
• R₄ darstellt
• eine Gruppe-CH(X)-O-C(O)-Y, wobei X und Y unabhängig voneinander eine Gruppe R₉ oder eine Phenylgruppe darstellen,
• eine Gruppe-CH₂CH₂-S-C(O)-W, wobei W eine Gruppe R₉ oder eine Phenylgruppe darstellt,
• R₅ darstellt
• eine Phenylgruppe, eine Benzylgruppe, die über die Phenylgruppe durch ein Halogenatom, eine Alkylgruppe mit 1 bis 4 Kohlenstoffatomen, eine Hydroxylgruppe, eine Alkoxygruppe -OR₉ oder eine Alkylsulfanylgruppe -SR₉ substituiert sein können,
• eine Biphenylmethylgruppe,
• R₆ eine Alkylgruppe mit 1 bis 6 Kohlenstoffatomen darstellt, die durch eine Hydroxylgruppe, eine Alkoxygruppe -OR₉, eine Sulfanylgruppe oder eine Alkylsulfanylgruppe -SR₉ substituiert sein können,
• R₈ darstellt
• eine Alkylgruppe mit 1 bis 6 Kohlenstoffatomen,
• eine Phenylgruppe, eine Benzylgruppe.

3. Verfahren zum Herstellen der Verbindungen der Formel (Ia) 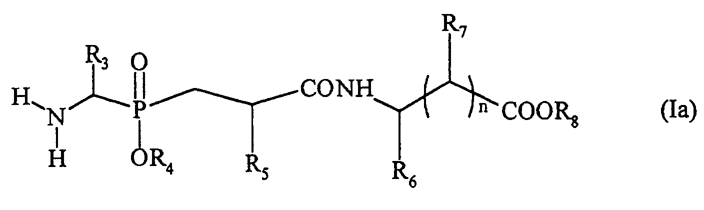 in welcher R₃, R₄, R₅, R₆, R₇, R₈ und n wie in Anspruch 1 definiert sind, das besteht aus
- Behandeln einer Verbindung der Formel (IVb) 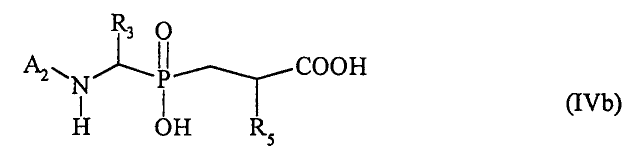
mit einer Verbindung der Formel (V), welche Verbindung in der Form eines Salzes verwendet werden kann, wie etwa das Salz der p-Toluolsulfonsäure, 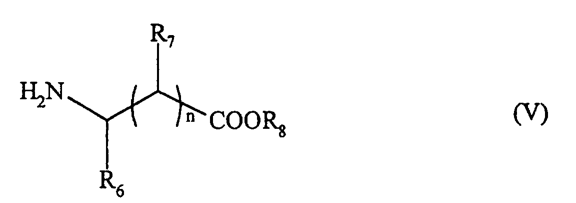 in welchen
R₃, R₅, R₆, R₇, R₈ und n wie in Anspruch 1 definiert sind und A₂ eine tert-Butoxycarbonylgruppe oder Fluorenylmethoxycarbonylgruppe darstellt, in Anwesenheit von Benzotriazol-1-yloxy-tris(dimethylamino)phosphoniumhexafluorphosphat (BOP) oder von 1-Ethyl-3-(3-dimethylamino-propyl)carbodiimidmethjodid (EDC), in einem organischen Lösungsmittel, wie etwa Dimethylformamid, um eine Verbindung der Formel (III) zu erhalten 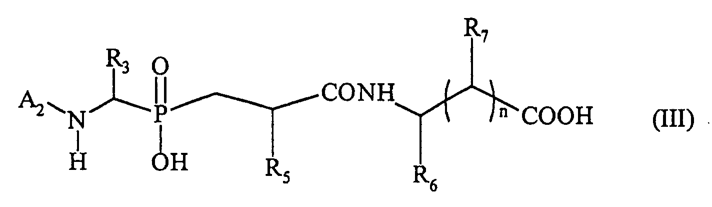 welche in einem organischen Lösungsmittel, wie etwa Tetrahydrofuran durch einen Alkohol der Formel R₄OH behandelt wird, in welchem R₄ so wie in Anspruch 1 definiert ist, in Gegenwart von N,N'-Dicyclohexylcarbodiimid und von 4-Dimethylaminopyridin, um die Verbindung der Formel (II) zu erhalten 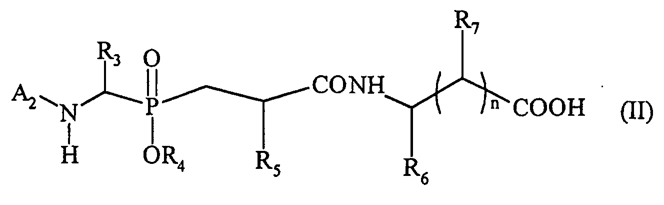 dann Schutzgruppenentfernung an der Aminofunktion, um eine Verbindung der Formel (Ia) zu erhalten, wobei der Schritt der Schutzgruppenentfernung in einem leicht sauren Milieu ausgeführt wird, wie etwa Ameisensäure, wenn die Gruppe A₂ eine tert-Butoxycarbonylgruppe darstellt, oder in einem basischen Milieu, wie etwa das Pyridin, wenn die Gruppe A₂ eine Fluorenylmethoxycarbonylgruppe darstellt, oder
- Umwandeln einer Verbindung der Formel (III), so wie vorstehend erhalten, in ein Cäsiumsalz durch Wirkung von Cäsiumcarbonat, und Behandeln des Salzes durch ein halogeniertes Derivat R₄Z, wobei Z ein Halogenatom, wie etwa Brom, Chlor oder Jod, darstellt und R₄ so wie vorstehend definiert ist, um die Verbindung der Formel (II) zu erhalten, dann Schutzgruppenentfernung der Aminfunktion, um eine Verbindung der Formel (Ia) zu erhalten, wobei der Schritt der Schutzgruppenentfemung in einem leicht sauren Mileu ausgeführt wird, wie etwa Ameisensäure, wenn die Gruppe A₂ eine tert-Butoxycarbonylgruppe darstellt, oder in einem basischen Milieu, wie etwa Pyridin, wenn die Gruppe A₂ eine Fluorenylmethoxycarbonylgruppe darstellt.

4. Verfahren zum Herstellen der Verbindungen der Formel (Ib) 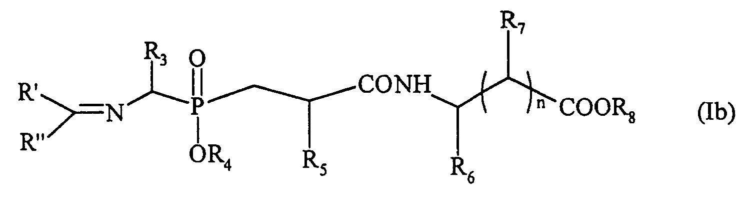 in welcher R', R", R₃, R₄, R₅, R₆, R₇, R₈ und n wie in Anspruch 1 definiert sind, das daraus besteht, ein Keton R'(R")C=O auf eine Verbindung der Formel (Ia) zu kondensieren, wie etwa in dem Anspruch 3 hergestellt, wobei die Ketone R'(R")C=O durch Fries-Umlagerung der entsprechenden Ester R"CO₂R' erhalten wurden.

5. Medikament, das **dadurch gekennzeichnet ist, dass** es durch eine Verbindung der Formel (I) gemäß Anspruch 1 oder 2 zusammengesetzt ist, wobei das Medikament zur Behandlung von beliebigen depressiven Zuständen, Schlafstörungen, Angststörungen, kognitive Störungen und Wachsamkeitsstörungen und periphere Störungen, wie etwa Diarrhoe, Husten und Entzündung, bestimmt ist.

6. Pharmazeutische Zusammensetzung, **dadurch gekennzeichnet, dass** sie eine Verbindung gemäß Anspruch 1 oder 2 in Verbindung mit einer beliebigen geeigneten Grundmasse enthält.

## Claims

1. A compound of the formula (I) 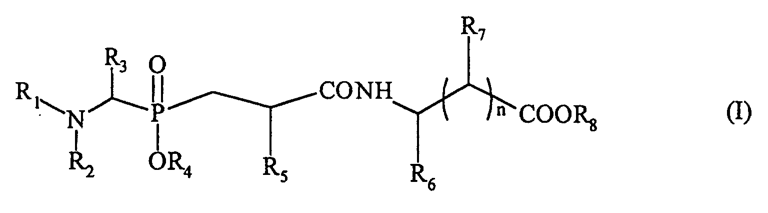 in which
• R₁ and R₂ each represent a hydrogen atom or R₁ and R₂, taken together, form an unsaturated group of the formula R'(R")C=, in which,
• R' represents a phenyl group in position 2 monosubstituted with a hydroxy group or a disubstituted phenyl group, in position 2, with a hydroxy group and, in position 4 or 5, either by a halogen atom or by a nitro group, or by a hydroxy group, or by an alcoxy group -OR₉,
• R" represents a phenyl group, a phenyl group substituted with 1 to 5 halogen atoms or an aromatic heterocyclic group,
• R₃ represents
• a hydrogen atom,
• an alkyl group or an alkenyl group of 1 to 6 carbon atoms, these two latter groups being liable to be substituted by:
• a hydroxy group or an alcoxy group -OR₉,
• a phenyl group or a benzyl group,
• a sulfanyl group, an alkylsulfanyl group -SR₉ or an alkylsulfanyl group oxidized at the sulfur atom -S(O)R₉,
• an amino group, an -NHR₉ group or -NR₉R₁₀ group, if desired oxidized at the nitrogen atom or,
• a guanidino group H₂N-C(=NH)-NH-,
• a cycloalkyl or cycloalkylmethyl group,
• a phenyl group, a benzyl group, which can be substituted on the phenyl group with 1 or 2 of the following substituents:
• a halogen atom,
• a hydroxy group, an alcoxy group -OR₉,
• an alkylsulfanyl group -SR₉ or an alkylsulfanyl group oxidized at the sulfur atom,
• an amino group or a -NHR₉ or -NR₉R₁₀ group if desired oxidized at the nitrogen atom,
• a nitro group,
• a phenyl group,
• an alkyl group of 1 to 4 carbon atoms,
• a methyl group substituted by an aromatic or saturated heterocyclic group, the heteroatoms being possibly oxidized in the form of N-oxide or S-oxide,
R₄ represents
• a -CH(X)-O-C(O)-Y group, in which X and Y represent, independently of each other, an R₉ group or a phenyl group,
• a -CH₂CH₂-S-C(O)-W group, in which W represents an R₉ group or a phenyl group,
R₅ represents
• a hydrogen atom,
• an alkyl group or an alkenyl group of 1 to 6 carbon atoms, these two latter groups being liable to be substituted with:
• a hydroxy group or an alcoxy group -OR₉,
• a phenyl group or a benzyl group,
• a sulfanyl group, an alkylsulfanyl group -SR₉ or an alkylsulfanyl group oxidized at the sulfur atom -S(O)R₉,
• an amino group, an -NHR₉ or -NR₉R₁₀ group, possibly oxidized at the nitrogen atom or,
• a guanidino group H₂N-C(=NH)-NH-,
• a cycloalkyl or cycloalkylmethyl group,
• a phenyl group, a benzyl group, which can be substituted at the phenyl group by 1 or 2 of the following substituents:
• a halogen atom,
• a hydroxy group, an alcoxy group -OR₉,
• an alkylsulfanyl group -SR₉ or an alkylsulfanyl group oxidized at the sulfur atom,
• an amino group or an -NHR₉ or -NR₉R₁₀ group which can be oxidized at the nitrogen atom,
• a nitro group,
• a phenyl group,
• an alkyl group of 1 to 4 carbon atoms,
• a methyl group substituted with a heterocyclic group, the hetero atoms being liable to be oxidized in the form of N-oxide or S-oxide,
• R₆ and R₇ represent independently of each other
• a hydrogen atom,
• an alkyl or alkenyl group of 1 to 6 carbon atoms, which can be substituted with:
* a hydroxy or an alcoxy group -OR₉,
* a sulfanyl group, an alkylsulfanyl group -SR₉ or an alkylsulfanyl group oxidized at the sulfur atom -S(O)R₉,
* an amino group or an alkylamino group -NHR₉,
* a guanidino group H₂N-C(=NH)-NH- or,
* a carboxy group or an alkyloxycarbonyl group -COOR₉,
• a phenyl group, a benzyl group, which can be substituted on the phenyl group by 1 or 2 of the following substituents:
* a halogen atom,
* a phenyl group,
* a hydroxy group or an alcoxy group -OR₉,
* an alkylsulfanyl group -SR₉ or an alkylsulfanyl group oxidized at the sulfur atom -S(O)R₉,
• R₆ and R₇ together represent a saturated or unsaturated cyclic compound of 5 or 6 members, comprising 1 or 2 heteroatoms selected from oxygen, sulfur and nitrogen,
• R₈ represents
• an alkyl or alkenyl group of 1 to 6 carbon atoms,
• a phenyl group, a benzyl group,
• R₉ and R₁₀ each represent an alkyl group of 1 to 6 carbon atoms, and
• n is equal to 0 or 1,
in the form of optical isomers, namely in the form of enantiomers and diastereoisomers and of mixtures of these different forms, including racemic mixtures as well as their pharmacologically acceptable acid addition salts, in which R₁ and R₂ are hydrogen atoms.

2. Compound according to claim 1, **characterised in that**
• R₁ and R₂ represent hydrogen atoms,
• n is equal to 0,
• R₃ represents
• a hydrogen atom,
• an alkyl group of 1 to 6 carbon atoms, which can be substituted with a hydroxy group, an alcoxy group -OR₉, a sulfanyl group or an alkylsulfanyl group -SR₉,
• a phenyl group, a benzyl group which can be substituted on the phenyl group with a halogen atom, an alkyl group of 1 to 4 carbon atoms, a hydroxy group, an alcoxy group -OR₉ or an alkylsulfanile group -SR₉,
• R₄ represents
• a -CH(X)-O-C(O)-Y group, wherein X and Y represent, independently of each other, an R₉ group or a phenyl group,
• a -CH₂CH₂-S-C(O)-W group, wherein W represents an R₉ group or a phenyl group,
• R₅ represents
• a phenyl group, a benzyl group, which can be substituted on the phenyl group with a halogen atom, an alkyl group of 1 to 4 carbon atoms, a hydroxy group, an alcoxy group -OR₉ or an alkylsulfanyl group -SR₉,
• a biphenylmethyl group,
• R₆ represents an alkyl group of 1 to 6 carbon atoms, which can be substituted with a hydroxy group, an alcoxy group -OR₉, a sulfanyl group or an alkylsulfanyl group -SR₉,
• R₈ represents one of the following:
• an alkyl group of 1 to 6 carbon atoms,
• a phenyl group, a benzyl group.

3. Process for the preparation of compounds of the formula (Ia) 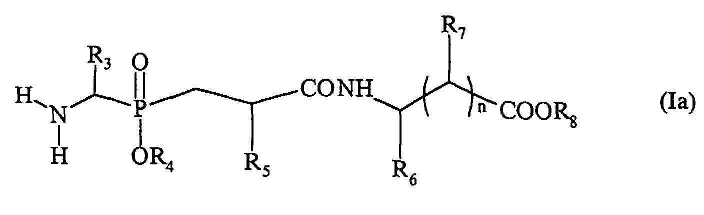 in which R₃, R₄, R₅, R₆, R₇, R₈ and n are as defined in claim 1, which consists:
- in contacting a compound of the formula (IVb) 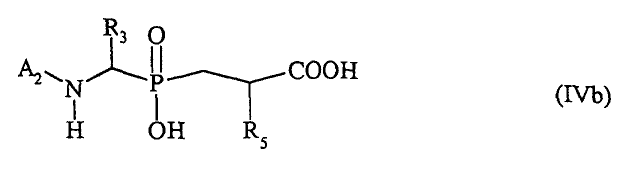
with a compound of the formula (V), which compound can be used in the form of a salt, such as the salt of *p*-toluenesulfonic acid, 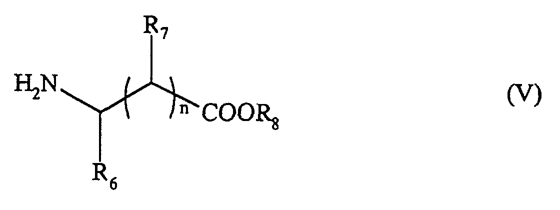 in which
R₃, R₅, R₆, R₇, R₈ and n are as defined in claim 1, and A₂ represents a *tert* -butoxycarbonyle group or a fluorenylmethoxycarbonyl group, in the presence of benzotriazol-1-yloxy-tris(dimethylamino)phosphonium hexafluorophosphate (BOP) or with 1-ethyl-3-(3-dimethylaminopropyl)carbodiimide methiodide (EDC), in an organic solvent such as dimethylformamide, to obtain a compound of the formula (III) 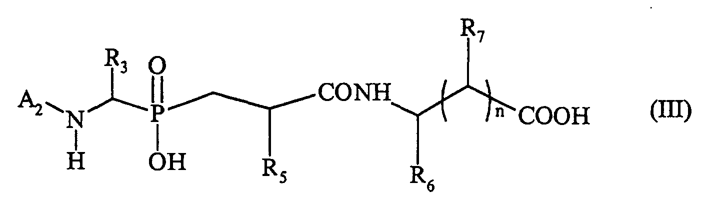 which compound is contacted, in an organic solvent such as tetrahydrofuran, with an alcohol of formula R₄OH, in which R₄ is as defined in claim 1, in the presence of *N*,*N*'-dicyclohexylcarbodiimide and 4-dimethylaminopyridine to obtain a compound of the formula (II) 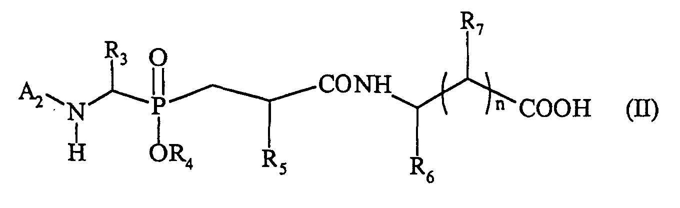 then removing the protective group of the amine function to obtain a compound of formula (Ia), the removal of the protective group being carried out in a weak acid medium, such as formic acid, if the group A₂ represents a tert-butoxycarbonyl group, or in a basic medium such as piperidine, if the group A₂ represents a fluorenylmethoxycarbonyl group, or
- transforming a compound of formula (III) as obtained as before into a cesium salt by the action of cesium carbonate, and contacting this salt with a halogenated derivative R₄Z, in which Z represents a halogen atom selected from the group consisting of bromine, chlorine or iodine, and R₄ is as defined above, to obtain the compound of formula (II), then removing the protective function of the amine group to obtain a compound of formula (Ia), the removal of the protective group being carried out in a weak acid medium, such as formic acid, if the group A₂ represents a tert-butoxycarbonyl group, or in a basic medium such as piperidine, if the group A₂ represents a fluorenylmethoxycarbonyl group.

4. Process for the preparation of compounds of formula (Ib) 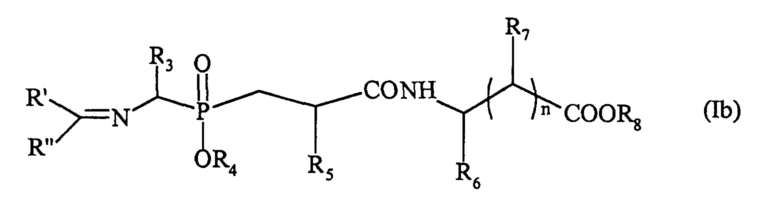 in which R', R", R₃, R₄, R₅, R₆, R₇, R₈ and n are as defined in claim 1, which consists in condensing a ketone R'(R")C=O on a compound of formula (Ia), as prepared in claim 3, these ketones R'(R")C=O being obtained by a Fries rearrangement of the corresponding esters R"CO₂R'.

5. A drug **characterised in that** it contains a compound of formula (I) according to claim 1 or 2, said drug being used for the treatment of depressive conditions of all nature, sleep disorders, anxiety disorders, cognitive and vigilance disorders, and peripheral disorders such as diarrhea, coughing and inflammation.

6. A pharmaceutical composition **characterised in that** it contains a compound according to claim 1 or 2, in association with any suitable excipient.
